# EUROPEAN PATENT SPECIFICATION

(11) **EP 2 365 984 B1**
(45) Date of publication and mention of the grant of the patent: **14.12.2016**
(21) Application number: 09786696.6
(22) Date of filing: 24.07.2009
(51) Int. Cl.: C07K 14/47

(54) **SYNDECAN-4 IS A REGULATOR OF RAC1-GTP**
SYNDECAN-4 IST EIN REGULATOR VON RAC1-GTP
SYNDÉCANE-4, RÉGULATEUR DE RAC1-GTP

(30) Priority: 24.07.2008 HU 0800464
(43) Date of publication of application: 21.09.2011
(73) Proprietor: Szilak Laboratories Bioinformatics & Molecule-Design Ltd., 6723 Szeged (HU)
(72) Inventor: SZILÁK, László, H-6723 Szeged (HU); KELLER-PINTÉR, Anikó, H-6640 Csongrád (HU); LETOHA, Tamás, H-6725 Szeged (HU); TÍMÁR, József, H-1112 Budapest (HU)
(74) Representative: Lengyel, Zsolt
(86) International application number: PCT/IB2009/053218
(87) International publication number: WO 2010/010532

(56) References cited:
- WO-A1-00/27416
- WO-A2-2008/010162
- BASS M D ET AL: "Cytoplasmic interactions of syndecan-4 orchestrate adhesion receptor and growth factor receptor signalling" BIOCHEMICAL JOURNAL, THE BIOCHEMICAL SOCIETY, LONDON, GB, vol. 368, no. 1, 15 November 2002 (2002-11-15), pages 1-15, XP002279782 ISSN: 0264-6021
- CARDIOVASCULAR RESEARCH, vol. 78, no. 2, May 2008 (2008-05), pages 223-231, XP002554142 ISSN: 0008-6363
- KOO B K ET AL: "Structural Basis of Syndecan-4 Phosphorylation as a Molecular Switch to Regulate Signaling" JOURNAL OF MOLECULAR BIOLOGY, LONDON, GB, vol. 355, no. 4, 27 January 2006 (2006-01-27), pages 651-663, XP024950595 ISSN: 0022-2836 [retrieved on 2006-01-27]
- MERTENS A E ET AL: "Regulation of Tiam1-Rac signalling" FEBS LETTERS, ELSEVIER, AMSTERDAM, NL, vol. 546, no. 1, 3 July 2003 (2003-07-03), pages 11-16, XP004433613 ISSN: 0014-5793
- JOURNAL OF CELL BIOLOGY, vol. 186, no. 1, July 2009 (2009-07), pages 75-83, XP002554143 ISSN: 0021-9525

## Description

The present invention provides a method for modulating the activity of Rac1, comprising modifying the phosphorylation state of Ser179 in syndecan-4 through influencing the interaction between syndecan-4 and the guanin nucleotide exchange factor of Rac1 (RacGEF) named Tiam1. In particular, methods are provided for enhancing the epithelial polarization of a cell by increasing the de-phosphorylation of said Ser179 of syndecan-4, and for enhancing the planar cell polarity subsequently migratory phenotype of a cell and for initiating the neuronal differentiation of a cell by increasing the phosphorylation of said Ser179 of syndecan-4. Polypeptides for use in modulating the activity of the small GTPase Rac are also provided, as well as a method for identifying an agent capable of indirectly modulating the activity of the Rac1 and consequently other small GTPases.

Small GTPases are molecular switches that use a simple biochemical strategy to control complex cellular processes. They toggle between two states: GTP-bound active and GDP-bound inactive. They common feature is hydrolyzing GTP to GDP. The Ras super family of small GTPases can be further divided into five major groups: Ras, Rho, Rab, Arf, Ran.

The Rho family has 12 members with their isoforms (Rho (A, B, C); Rac1,2,3; Cdc42; TC10; TCL; Chp(1,2); RhoG; Rnd(1,2,3); RhoBTB(1,2); RhoD,; Rif; TTF) among them the three best characterized are RhoA, Cdc42, Rac1 Their regulation happens very precisely. The human genome contains over 60 activators (guanine nucleotide exchange factors (GEFs)) catalyzing the GTP exchange, and more then 70 inactivators (GTPase- activating proteins, GAPs) stimulating GTP hydrolysis, leading to inactivation for this family. There are four GTP exchange inhibitors (GDIs) blocking the activation circle (Etienne- Mannville and Hall, 2002).

The small GTPases have two stages of activity: an inactive GDP-bound conformation and an active GTP-bound conformation. In their active conformation they interact with effector proteins, which induce downstream signaling events. The GDP-GTP cycle is highly regulated by guanine nucleotide exchange factors (GEFs) that induce the release of the bound GDP to be replaced by GTP. The small GTPases can hydrolyze GTP by intrinsic GTPase activity slowly but it is stimulated further by GTPase-activating proteins (GAPs) (Bos et al., 2007). The GTP-loading cycle is regulated by guanin dissociation inhibitors (GDIs), which binds the hydrolyzed form of GDP-small GTPase preventing a GEF stimulated GTP exchange (Dransart et al., 2005).

The regulation of the GTP-GDP cycle can occur via direct perturbation of GEFs. Brefeldin A, a compound produced by a fungal organism inhibits an ArfGEF (Sec7) resulting in the collapse of the Golgi structure. Brefeldin A stabilizes the ternary complex of Arf-GDP-GEF thus traps the GEF in an unproductive complex with its substrate. The compound NSC23766 was identified as an inhibitor of the interaction between Rac1 and Tiam1, a RacGEF (Gao et al., 2004). For certain GEFs such as EPAC proteins, activation rather than inhibition may have therapeutic benefits because they are involved in the formation of cell-cell junctions. Junctions are critically important for the integrity of epithelial and endothelial monolayers (Bos et al., 2007).

The apical-basal polarity of mammalian epithelial cells is established and maintained through the formation of specialized intracellular junctions, the adherens junctions (AJs) and the tight junctions (TJs). On the formation of initial intercellular contacts by cadherins (known as primordial adhesions), the activation of the Par polarity complex by Cdc42 and/or Rac1 is required for the establishment of TJs. Par3-Par6-aPKC localize to primordial TJs and regulate their maturations and positioning with respect to to basolateral and apical membrane domains. Tiam1 a RacGEF directly associates with the Par polarity complex by binding to Par3, thereby controlling TJ assembly in epithelial cells. Tiam1-deficient cells are impaired in TJ maturation. Disrupting or delocalization of the complex of Tiam1-Rac1-Par3-Par6-aPKC is a crucial step in the process of the epithelial-mesenchymal transition (EMT), a requirement for tumour cells to become invasive and metastasize (Mertens et al., 2003).

Rac1 Rho, Cdc42 are implicated in formation of morphology, cell polarity, motility and regulate three separate signal transduction pathways linking plasma membrane receptors to assembly of distinct filamentous actin structures. Their local distributions show characteristic patterns. Cdc42 activity is most prominent at the tip of the leading edge and it is known to trigger filopodium formation. The concentration of active Rac1 is gradually increasing toward the leading edge (Itoh et al., 2002; Ridley et al., 2003) and it induces plasma membrane protrusions as lamellipodia and membrane raffles by stimulating actin polymerization at the leading edge inducing recruitment and clustering of activated integrins there (Nobes and Hall, 1995; Ridley, 2006). The activity of Rho is elevated in the trailing edges implicated in the development of mature, more stable focal adhesions at the side and rear involved in the reorganization of actin stress fibers and myosin-induced contractility (Ridley, 2006).

The activity of Rho family is directly influences the tissue development. Beyond the epithelial phenotype the neuronal differentiation is regulated by Rac, Rho, Cdc42 through their effectors. In neuronal cells they orchestrate migration, neuritogenesis, growth cone stability and dendritic spine morphogenesis (Linseman and Loucks, 2008).

Syndecans constitute a family of four transmembrane heparan sulfate proteoglycans (HSPG) (Bernfield et al., 1992). They share a uniform structure: a divergent extracellular domain (ectodomain) containing glycosaminoglycan (GAG) attachment sites (Kokenyesi and Bernfield, 1994; Langford et al., 1998), a conserved one-span transmembrane domain (TM) and a conserved short (approximately 30 amino acids) cytoplasmic domain (CD).

Among the family members only syndecan-4 is expressed ubiquitously, involved directly in signal transduction and in the regulation of a variety of cellular processes.

The role of syndecan-4 in the regulation of Rac1-Cdc42 was previously reported. The Rac1 activity was elevated in Syndecan-4 null cells and in the mutation where last four amino acids EFYA, known as II type PDZ binding site were deleted (Saoncella et al., 2004, Tkachenko et al., 2004; 2006). In wild-type fibroblasts, syndecan-4 mediated the matrix induced PKCa -dependent activation of Rac1 (Bass et al., 2002). The migration of endothelial cells was dependent on the PDZ-binding site of syndecan-4. In the case of the lack of the syndeac-4 PDZ binding site the Rac 1 level was increased and the cells failed to migrate (Tkachenko et al., 2006).

Murakami et al (2008) review fibroblast growth factor signalling in angiogenesis. It is stated that in an unclustered state, syndecan-4 suppresses Rac1 activation and cell migration and thereby suppresses angiogenesis.

Elfenbein et al (2009) describe the signalling cascade from syndecan-4 after activation by FGF.

WO 00/27416 describes a method for stimulating angiogenesis by bringing ser183 of syndecan-4 into an non-phosphorylated state by inhibiting protein kinase C delta or increasing the amount of activated protein kinase C alpha.

WO 2008/010162 is an application by the same inventors and describes the use of syndecan-4 for modulating the intracellular targeting of an agent by modifying syndecan-4 of said cell.

However, the exact mechanism by which the syndecan-4 affects the regulatory pathway of Rac1 is not known. Therefore, there no real tool is available in the art to effectively modulate the activity of Rac1, and thus modulate the behavior of different type of cells, which might be desired as diagnostic or therapeutic targets.

The present inventors identified the underlying molecular mechanism in the syndecan-4 mediated Rac1 activity. This will allow the identification and application new compounds and composition to modulate the activity of Rac1 in cells. The implication of small GTP-ases in cancers are well known, e.g. Ras mutation was found in more than 15% of all human cancers (other examples takes it as 30%) (Bos et al., 2007), thus it can bear wide ranging potential in therapy of different clinical conditions.

The present invention is first to describe that the phosphorylation of the cytoplasmic serine of syndecan-4 could determine the activity of small GTPase Rac1 restricting the interaction of Rac1 with the RacGEF Tiam1 and regulating the Rac-GDP and Tiam1 interaction.

The implication of syndecans in the neuronal development (Ethel et al., 2001), more specially syndecan-4 were characterized in the state-of-art (Matthews et al., 2008; Kuriyama and Mayor, 2009), however the influence of the phosphorylation status of syndecan-4 for the neuronal development was not disclosed.

Accordingly, the present invention provides a polypeptide selected from the group consisting of the full length syndecan-4, and a fragment of human syndecan-4 cytoplasmic domain comprising the serine residue at position 179 (Ser179) of at least 13 amino acids in length, wherein said Ser179 residue of syndecan-4 is in phosphorylated state or modified to a phosphomimetic amino acid residue, for use in the treatment of a disease selected from the group consisting of cancer. Down syndrome, Wiskott-Aldrich syndrome, leukemia, ischemia or excitotoxicity, polyglutamine diseases, traumatic nerve injury, amyotrophic lateral sclerosis, neuropsychiatric disorder and neurodevelopmental disorders, wherein said polypeptide decreases the activity of Rac1.

In an embodiment, the cancer is selected from the group consisting or breast adenocarcinoma, colon carcinoma, gastric carcinoma, prostate carcinoma, endometrial carcinoma, melanoma.

In another embodiment, the neuropychiatric disorder is selected from the group consisting of schizophrenia, Alzheimer's disease, HIV encephalitis.

In another embodiment, the nettrodevelopmental disorder is selected from the group consisting of fragile X mental retardation syndrome, tuberous sclerosis complex, autism, Rett syndrome, sympathetic neuron death.

In another embodiment, the polypentide has the sequence of SEQ.ID.NO.: 3 or SEQ.ID.NO.: 4.

In another embodiment, the phosphomimetic amino acid residue is the result of a Ser179Glu modification.

The present invention also provides a polypeptide selected from the group consisting of the full length human syndecan-4, the cytoplasmic domain of human syndecan-4, and a fragment of human syndecan-4 cytoplasmic domain comprising the serine residue at position 179 (Ser179) of at least 13 amino acids in length, wherein said Ser179 residue of syndecan-4 is in dephosphorylated state or modified to a phosphodefective amino acid residue for use in the treatment of Chronic granulomatous disease (CGD), wherein said polypeptide increases the activity of Rac1.

In an embodiment, the the polypeptide has the sequence of SEQ.ID.NO.: 1 or SEQ.ID.NO.: 2.

In another embodiment, the phosphodefective amino acid residue is the result of a Ser179Ala modification.

### Detailed description

The present invention, in one embodiment, provides a polypeptide selected from the group consisting of the full length syndecan-4, the cytoplasmic domain of syndecan-4, a fragment of syndecan-4 cytoplasmic domain comprising the serine residue at position 179 (Ser179) and a functional equivalent derivative thereof, for use in modulating the activity of the small GTPase Rac1. In another corresponding embodiment, the present invention provides a method for modulating the activity of Rac1, comprising modifying the phosphorylation state of Ser179 in syndecan-4.

The small GTPase Rac1 plays central role in determination of the epithelial phenotype. The Rac1-GTP level was increased 2-4-fold upon induction of intercellular adhesion (Betson et al., 2002; Bass et al., 2002). With Ca²⁺ switch method 3.7±0.9-fold increment of Rac1 activity was measured in MDCK cells and 1.9±0.4-fold increase in HEK293 cells, but practically there was no change in NIH3T3 fibroblasts (Noren et al., 2001). The potential role of syndecan-4 in the regulation of the active Rac1 level was previously proposed because in syndecan-4 null (-/-) cells the Rac1-GTP level was elevated 3.6-fold or 2.3-fold (Saoncella et al., 2004; Bass et al., 2002). Clustering of syndecan-4 or lacking the PDZ binding site of syndecan-4 cytoplasmic domain also elevated the basal levels of Rac1 blocking the migration of endothelial cells (Tkachenko et al., 2004; 2006).

Accordingly, the term "activity of the small GTPase Rac1" refers to any activity of Rac1. Non-limiting examples of Rac1 activity include the activities expressely mentioned herein. In particular, Rac1 interact with various effector proteins, for example, with S6 kinase (translation regulation), MLK2/3 (JNK kinase), MEKK1/4 (JNK), PAK1/2/3 (LIMK kinase actin cytoskeleton), PI-4 kinase (PIP2), PI3K (PIP3), DAG (PA levels), PLD (PA levels), WAVE/SCAR (actin cytoskeleton), POR1 (actin cytoskeleton), IQGAP1/2 (cell-cell contact).

The term "modulation" refers to either up or down-regulation of the activity of Rac1, including activation, inhibition, complete or partial blocking of said activity. The level of modulation may vary in wide ranges, but usually up-regulation results in at least 20%, preferably at least 30%, 40% more preferably 50% increase in the activity of Rac1 compared to the normal activity level. Conversely, in case of down-regulation, the activity of Rac1 decreases by at least 20%, preferably at least 30%, 40% more preferably 50% the activity of Rac1 compared to the normal activity level.

In the context of the present description, the term polypeptide is intended to mean polypeptides of any length. In particular, basic polypeptides as short as 5 amino acids are capable to specifically bind to syndecan-4 ands influence its phosphorylation state. There is no particular limitation on the origin of the polypeptides, the may be synthesized according to techniques well-known to the person skilled in the art, or may be isolated from natural sources, or produced by biotechnological means. The size of the polypeptide may be a deciding factor on the origin, smaller peptides may be easily synthesized by chemical means, but their preparation by recombinant means might be problematic, and vice versa. The person skilled in the art will readily determine the optimal source of the polypeptide of the invention according to the requirements of the task at issue.

As used herein, the term "syndecan-4" refers to the syndecan-4 from any origin, preferably mammalian, more preferably human origin.

As used herein, the term "cytoplasmic domain of syndecan-4" refers to the polypeptide comprised of the amino acid residues 170 to 199 of syndecan-4. The cytoplasmic domain of syndecan-4 comprises one serine residue at position 179 (Ser179), the phosphorylation state of which determines, according to the present invention, the activity of Rac1 through interaction of with Tiam-1. Tiam-1, as referred to herein, is a guanin nucleotide exchange factor of Rac1 (RacGEF). The interaction is GTP-dependent.

The term "phosphorylation state of Ser179" refers to the serine residue at the position 179 in syndecan-4 amino acid sequence being either phosphorylated or not. The term "modifying" the phosphorylation state of Ser179 includes either the phosphorylation of the residue from non-phosphorylated state, or the de-phosphorylation of the residue from phosphorylated state.

In certain embodiments, the invention may be carried out by using a fragment of syndecan-4 cytoplasmic domain comprising the serine residue at position 179 (Ser179). In this respect, there is no structural limitation to the fragment, as long as the Ser179 is capable of being phosphorylated, and further capable of interaction with Tiam-1 to influence the activity of Rac1.

In addition, the present invention may utilize a functional equivalent derivative of these polypeptides. The term "functional equivalent derivative of a polypeptide" means that the said functional equivalent derivative substantially performs the same way when placed under substantially equivalent conditions as said polypeptide, i.e. has substantially the same activity, affinity, reaction kinetics towards the ligands or reaction partners of said polypeptide. According to this definition, the polypeptide of the invention may or may not comprise the Ser179 of syndecan-4 in its native sequence, i.e. within the context of the amino acids occurring in the native syndecan-4.

In a specific embodiment, the functional equivalent according to the invention comprises a subsequence of syndecan-4 lacking the Ser179 position, i.e. the whole polypeptide spans amino acid residues of syndecan-4 entirely before the 179 position, or entirely after the 179 position. This functional equivalent polypeptide has the effect of a non-phosphorylated polypeptide according to the invention, and may up-regulate the activity of Rac1.Without wishing to limit the invention by theory, it may be viewed as a phosphorylation antagonist.

The length of the polypeptide of the invention can vary in a wide range. Peptides as short as 13 amino acids in length (SEQ.ID.NO.: 2) may fall under the scope of the present invention, or even up to the full length syndecan-4. However, the length of the polypeptide is not limiting for the invention, as long as it has the necessary activity to modulate the activity of Rac1.

In a further specific embodiment, the functional equivalent is capable to induce the phosphorylation of syndecan-4, without being a structural homologue of syndecan-4. Therefore, such polypeptides are specifically included within the scope of the present invention, as long as their effect on the cell, even if only indirectly, results in the modulation of the activity of Rac1. Ultimately, the functional derivative may or may not be a peptide, it may be any biomolecule, or even smaller compounds capable to influence the phosphorylation state of syndecan-4.

In a preferred embodiment, the polypeptide is modified at the Ser179 position to either to a phosphodefective (i.e. non-phosphorylable) or a phosphomimetic amino acid residue. The term "phosphodefective or non-phosphorylable" refers to a modification wherein the amino acid residue substituted at position 179 is not suitable to being phosphorylated. Conversely, the term "phosphomimetic" refers to a substitution wherein the amino acid residue substituted mimics a phosphorylated serine residue without being phosphorylated, to avoid the possibility of de-phosphorylation.

The person skilled in the art will be readily able to determine the nature of the necessary amino acid substitutions. In specific embodiments, the phosphodefective amino acid residue modification is Ser179A1a. Accordingly, this modification results in the up-regulation of Rac1 activity. In other specific embodiments, the phosphomimetic amino acid residue modification is Ser179Glu, and the modification results in the down-regulation of Rac1 activity. Said modifications may be present on either on the full length syndecan-4, on the cytoplasmic domain of syndecan-4, on a fragment of syndecan-4 cytoplasmic domain comprising the serine residue at position 179 (Ser179) or on a functional equivalent derivative thereof.

In an embodiment of the present invention, the polypeptide according to the invention comprises the Ser179 in its phosphorylated state or comprises a phosphomimetic residue in the syndecan-4 sequence, which in turn decreases the activity of Rac1 In another embodiment, the polypeptide according to the invention comprises the Ser179 in its de-phosphorylated state or comprises a phosphodefective residue in the syndecan-4 sequence, which in turn increases the activity of Rac1. In related embodiments, the present invention provides methods, wherein the phosphorylated state of said Ser179 of syndecan-4 is enhanced, thereby the activity of Rac1 decreased, or conversely, wherein the non-phosphorylated state of said Ser179 of syndecan-4 is decreased, thereby the activity of Rac1 is increased.

According to our observation, the expression of Ser179Ala syndecan-4 resulted in significant increment of the level of activated Rac1, while the Ser179Glu syndecan-4 production resulted in the depletion of active Rac1. The elevation of the active Rac1 level might come either from a missing interaction of Ser179Ala protein similarly to the syndecan-4 null case or Ser179A1a itself would enhance the Rac1-GTP synthesis. Since the antiserum raised against the Rac1-specific guanine nucleotide exchange factor (RacGEF) Tiam-1 precipitated all syndecan-4 variants and preferred the Ser179A1a mutant the most in GDP saturated lysate, although we do not intend to limit the invention by theory, the enhancer theory is preferable. On the contrary, when the cell lysate was saturated with γS-GTP the Ser179Glu was preferably co-precipitated. Most probably the Ser179Ala mediates binding of Tiam-1 and Rac1-GDP accelerating the production of Rac1-GTP. The binding of Ser179Glu to Tiam-1 must block somehow the enzyme activity because the expression of Ser179Glu decreased the Rac1-GTP level 1.5-fold. Since Rac1 failed to be detected in the anti Tiam-1 immunoprecipitation in the Ser179Glu expressing cell lysate we can conclude that the Ser179Glu syndecan-4 can interfere with the binding of Rac1-GDP to Tiam-1.

In another embodiment of the present invention, a method is provided for enhancing the epithelial phenotype of a cell, comprising increasing the de-phosphorylation of said Ser179 of syndecan-4.

Tiam-1 and Rac1 initiate epithelial polarity interacting with the Par polarity complex formed by Par3, Par6 and aPKC. Loss of apical - basal polarity in epithelial tissue is a crucial step in the progress of the epithelial-mesenchymal transition (EMT) (Mertens et al., 2003). The expression of Ser179Glu syndecan-4 beyond decreasing the concentration of Rac1-GTP can interfere with the formation of the proper Tiam - Par complex, which can promote EMT (Mertens et al., 2003).

Otherwise, a direct binding of syndecan-4 to Tiam-1 can also be considered because Tiam-1 contains CASK/Lin2 type PDZ binding domain (Songyang et al., 1997) that was shown to bind the conserved PDZ binding site of syndecans (EFYA) (Hsueh and Sheng, 1999). In line with that, our results can be interpreted that strength of the interaction between syndecan-4 and Tiam-1 depends on the phosphorylation state of Ser179. The phospho-mimetic Ser179Glu mutant was enriched in Tiam-1 immunoprecipitates in the presence of GTP and the Ser179Ala mutant was more abundant in the GDP saturation. The increment of the activated Rac1 level in the case of the lack of the PDZ binding site EFYA (Tkachenko et al., 2006) can be similar to the syndecan-4 null case (Saoncella et al., 2004) because the syndecan-4 - Tiam-1 interaction cannot occur contrary to Ser79Ala, where EFYA was present.

In another embodiment of the present invention, a method is provided for initiating the polarization of a cell, comprising increasing the de-phosphorylation of said Ser179 of syndecan-4.

Tiam-1 and Rac1 initiate epithelial polarity interacting with the Par polarity complex formed by Par3, Par6 and aPKC. Loss of apical - basal polarity in epithelial tissue is a crucial step in the progress of the epithelial-mesenchymal transition (EMT) (Mertens et al., 2003). The expression of Ser179Glu syndecan-4 beyond decreasing the concentration of Rac1-GTP can interfere with the formation of the proper Tiam - Par complex, which can promote EMT (Mertens et al., 2003).

Administration ofphorbol-12-myristate-13-acetate (PMA) stimulated solitary cell migration in nt-MCF-7 cells, wt-syndecan-4 and sheet migration in Ser179Ala expressing lines with similar velocity (0.3 µm/min) to that measured in the untreated Ser179Glu syndecan-4 expressing cells. The movement was accompanied with reduced level of Rac1-GTP. However, administration of PMA improved the epithelial morphology of Ser179Glu syndecan-4 cells with concomitant increase of Rac1-GTP up to the level of non-treated wt-syndecan-4, accompanied by recruitment of vinculin to the focal adhesions. Restoration of the epithelial character of the Ser179G1u cells upon PMA administration can be explained by that the DAG analogue PMA might compensate the perturbation caused by Ser179Glu syndecan-4.

Without being limited by theory, these observations suggest that the syndecan-4 forms studied here affected the concentration of Rac1-GTP through controlling the activity of Tiam-1, which in turn altered the phenotype of the lines. The Ser179Ala mutation elevated the concentration of Rac1-GTP along with enhanced epithelial polarization, whereas Ser179Glu mutation decreased the active Rac1 concentration accompanied by cell migration.

We and others were able to show that syndecan-4 is implicated in the regulation of Rac1 activity thus, consequently, driving membrane protrusions and the formation of nascent focal complexes and actin - filament bundling (Tkachenko et al., 2004, Mostafavi-Pour et al., 2006, Tkachenko et al., 2006; Bass et al., 2002). However, our finding that Ser179Glu syndecan-4 downregulated and the Ser179Ala mutant increased the Rac1-GTP level may contribute to the decreasing Rac1 gradient from the leading edges towards the trailing edges (Itoh et al., 2002; Burridge and Doughman, 2006) because the Ser179Ala syndecan-4 was characteristic for the leading edges, filipodia, and lamellipodia while Ser179Glu was accumulated in the trailing edges distributed along the actin stress fibers and in the focal adhesions. The activity of Rac1 and Rho is mutually exclusive (Sander et al., 1999; Ohta et al., 2006) and spatially distinct in a migrating cell: at the leading edges Rac1 activity is dominating while Rho activity is gradually increasing toward the trailing edge (Itoh et al., 2002; Ridley et al., 2003).

Despite the early expectations that the non-phosphorylated syndecan-4 might prefer binding to α-actinin distributed in the focal adhesions (Longley et al., 1999; Greene et al., 2003; Couchman, 2003; Dovas et al., 2006), the phospho-syndecan-4 was shown to bind with high affinity to α-actinin (Chaudhuri et al., 2005). Indeed, only the Ser179Glu syndecan-4 was concentrated in the focal adhesions and distributed abundantly along the actin cytoskeleton.

Overexpression of wild type, or GFP-tagged syndecan-4 did not raise the amount of syndecan-4 significantly in the focal adhesions in MCF-7 cells. The published data are split concerning the presence of syndecan-4 in the focal adhesions: the observation was dependent on the cell lines, the morphology of the cells, the spatial distribution of focal adhesions in the cells, and the epitopes of syndecan-4 antibodies (Woods and Couchman, 1994; Baciu and Goetinck, 1995; Greene et al., 2003). In syndecan-4 null (-/-) fibroblast smaller focal complexes were demonstrated (Ishiguro et al., 2000), most probably because of the elevated concentration of Rac1, which resulted in small focal complexes (Nobes and Hall, 1995).

According to our observation the expression of Ser179Ala syndecan-4 elevating Rac1 level resulted in the formation of small focal complexes. The Ser179Glu expressing cells developed mature focal adhesions accumulating Ser179Glu syndecan-4 and FAK but devoid ofvinculin. Despite the extensive studies on vinculin its precise role in focal adhesions is enigmatic. Vinculin is a focal adhesion component that can bind F-actin, PKCα, α-actinin and several other proteins (Ziegler et al., 2006; Hu et al., 2007). Downregulation, or elimination of vinculin from focal adhesions led to increased cell motility (Ziegler et al., 2006). If the recruitment of vinculin was blocked to the focal adhesions the migration velocity of MDA-MB231 cells was greatly increased (Torka et al., 2006).

The recruitment of vinculin to focal adhesions can be blocked directly or indirectly. 1) Direct blockade of vinculin recruitment would involve a competition of the Ser179Glu syndecan-4 for the same site e.g. for the α-actinin binding site. This theory is supported by the fact that the vinculin-positive focal adhesions formed in Ser179Glu cells upon PMA incubation seldom co-localized with Ser179Glu syndecan-4. 2) On the other hand, the presence of vinculin in focal adhesions must be modulated by syndecan-4 indirectly, e.g. via some protein kinases. It has been previously shown that inhibition of PKCα activity released vinculin from focal adhesions in REF cells (Dovas et al., 2006). We observed the same effect of the PKCa inhibitor Gö6976 on our MCF-7 cell lines. Taking together, the lack of vinculin from focal adhesions can contribute to the migratory phenotype.

Epithelial cells are rich in cell-cell junctions, which have critical role in maintaining the epithelial morphology. The adherens junctions are composed of transmembrane cadherin proteins forming a continuous adhesion belt (*zonulae adherens*) encircling each of the interacting cells in the sheet and in the meantime the cadherin cytoplasmic domain anchors the actin cytoskeleton through catenins (Yap et al., 1997). Rac1 and other small GTPases, and their effector enzimes are present at the cell-cell junctions. Overexpression of constitutively active Rac1 induced greater accumulation of E-cadherin, β-catenin and F-actin at sites of cell-cell contact; by contrast expression of dominant negative Rac1 inhibited their accumulations (Nakagawa et al., 2001; Ehrlich et al., 2002). The consequent E-cadherin clustering was supposed to trigger junctional actin assembly, which, thereafter appears as junctional actin forming a thicker, continuous F-actin belt at cell-cell borders of mature epithelia (Adams et al., 1998; Zhang et al., 2005). The maintenance and the formation of E-cadherin-mediated adhesions was promoted by Tiam1 (Sander et al., 1999). On the other hand, it was demonstrated that Rho effector Dia-driven actin polimerization stabilized the adherens junction complexes and promoted the localization of E-cadherin and α-catenin to the cell border, even in the outside lateral regions that not in contact with other cells (Sahai and Marshall, 2003). This outside E-cadherin ring might facilitate the formation of continuous, circumferential cortical actin ring around the peripheral cells of clusters, which was observed in Ser179Ala cells. Syndecan-4 can be implicated in the regulation of the cell-cell adhesions most probably with Rac1 activation via regulation of Tiam-1 activity. In fact, the Ser179Ala cells showed enhanced competence for cell-cell adhesion with improved epithelial character.

Beyond the previously classified epithelial actin fibers of thin actin rings at the bottom and junctional actin bundles in the apical region of the cells we also distinguishes a peripheral actin belt encircling the whole clusters. Since a cluster contains relatively few cells we think the phenomenon of the arch-like actin bundles is because of the flat cells at periphery, and taller in the middle of clusters. During the development of the epithelial polarization the average height of the cells should be leveled up and so the junctional actin bundles will be raised into the typical area of *zonulae adherens* close to the apical surface. Indeed, arch-like organization could not be seen in confluent culture, instead continuous network of junctional actin bundles. The organization of the junctional actin bundles in the apical region is distinct from the other organization of actin filament e.g. stress fibers on the basal surface and the thick circumferential actin ring around the clusters.

The virtually homogenous thick bundles around the clusters constituted from thin actin bundle vertically oriented underneath each other on the spherical cell surface. We surmise the circumstantial actin cable around the clusters is to constrain the cells to cells to force epithelial polarization inhibiting cells spreading.

Since the observed velocity of migration on fibronectin was approximately 0.3 µm/cm that is similar to the elongation rate of the actin polymer (Hotulainen and Lappalainen, 2006) probably the migration is purely mesenchymal (Friedl, 2004).

In another embodiment of the present invention, a method is provided for enhancing the migratory phenotype of a cell, comprising increasing the phosphorylation of said Ser179 of syndecan-4.

The importance of PKC activation in cell migration of MCF-7 cells has long been recognized (Johnson et al., 1999; Ng et al., 1999; Parsons et al., 2002; Torricelli et al., 2005). Overexpression of intact PKCα but neither the kinase-defective PKCα nor PKCa regulatory domain promoted migration of MCF-7 cells (Ng et al., 1999). In our wt- and Ser179Ala syndecan-4 lines PMA induced cell migration, however, not in the originally migratory Ser179Glu cells, which became epithelial.

It has been recently shown that following PMA incubation p130Cas, Crk and Rac1 translocated to the lamellipodia, triggering cytoskeletal reorganization that would be modulated via PKC/Src/p130Cas/Crk/Rac1 signaling pathway (Nomura et al., 2007). Therefore PKCa is an upstream activator of Rac1 because inhibition with Gö6976, a potent PKCa inhibitor resulted in reduced Rac1-GTP (Bass et al., 2002; Mendoza-Naranjo et al., 2006). Our data verified this observation because the level of activated Rac1 was reduced markedly following Gö6976 treatment of the lines. However, in the case of Ser179Ala the reduction did not access the level of GTP-Raclcharacteristic for the non-treated wt-syndecan-4 cells that is the Ser179Ala must act on independent pathway on the formation of Rac1-GTP.

The detected reduction of the active Rac1 in the case of Ser179Glu syndecan-4 cells cannot be merely the consequence of the lack of the PKCα - syndecan-4 interaction, since Tyr188Leu syndecan-4, which is also unable to interact with PKCα similarly to Ser179Glu (Lim et al., 2003) was incapable of changing the level of Rac1-GTP (Bass et al., 2002). This confirms the theory that syndecans are implicated in the regulation of the formation of the Rac1-GTP other than influencing the activity of PKCa e.g. influencing the activity of Tiam-1.

Further more, upon Gö6976 treatment a slow cell migration (0.05 μm/min) was observed in the wt- and Ser179Ala syndecan-4 cell lines that can argue the key role of PKCα in cell migration.

Depending on the cell lines, two types of cell migration were distinguished: Solitary cell or multi-cell sheet migration. The Ser179Glu syndecan-4 expressing cells were always migrating as individual cells. Upon PMA stimulation of the nt-MCF-7, or wt-syndecan-4 expressing lines solitary cells left the clusters and migrated alone, on the contrary Ser179Ala cells preferred migrating in clusters that is the establishment of the specialized intercellular junctions were essential. When an individually migrating Ser179Ala cell met a cluster, joined to it and forced the whole group to move with similar rate as the cell moved, meaning that (i) the cells expressing Ser179Ala syndecan-4 had high competence for the cell-cell adherence and (ii) the cluster was ready to migrate.

The motility of the quiescent MCF-7 cells upon expression of Ser179Glu raised the question whether epithelial-mesenchymal transformation (EMT) has occurred. Enhanced cell migration was concomitant with loss of E-cadherin from the cell contacts (data not shown) similarly to the published observations for mesenchymal cells (Adams, 2001, Ray et al., 2007). On the other hand, detection of vimentin, a widely accepted marker of mesenchymal cells (Thiery, 2003; Hay, 2005) was failed (data not shown). In fact, there are no outstanding specific biochemical markers by which the mesenchyme can be defined (Hay, 2005).

In another embodiment of the present invention, a method is provided for initiating the neuronal differentiation of a cell, comprising increasing the phosphorylation of said Ser179 of syndecan-4. The phosphorylation of Syndecan-4 at the position Ser179 results in the initiation of neuronal differentiation or shifts the state of a cell from a dynamic equilibrium towards a differentiated state into a neuronal type phenotype. Therefore, the present invention provides the potential for a major breakthrough in the treatment of many disorders and conditions characterized by the dysfunction or degeneration of neurons or nerves.

In another embodiment of the present invention, a method is provided for mediating the localization of Par polarity complex, comprising modifying the phosphorylation state of Ser179 in syndecan-4.

To establish the tight and adherens junctions (TJs and AJs) the activation of Par polarity complex is required by binding the active form of Rac1 and/or Cdc42 small GTPases. Par3, Par6 and atypical PKC (aPKC) localize to the primordial AJ and/ or TJ and regulate their positioning and maturation (Mertens et al., 2003; Suzuki and Ohno, 2006). Although both dominant-active Rac1 or Cdc42 was able to induce TJ formation yet the activity of Cdc42 is dispensable for apical-basal cell polarization (Mertens et al., 2003). The Rac1 activator Tiam-1 directly associates with the Par polarity complex by binding to Par3, but not to Par6. However, uniquely Par6 possesses a semi Cdc42/Rac1 binding motif (CRIB), which with the adjacent PDZ domain is capable of binding Cdc42/Rac1 that is required for biological activity of Par6 (Garrard et al., 2003). The N-terminus of Par6 forms a constitutive complex with an aPKC (λ/ζ) that is part of the polarity complex. The Tiam-1- Par3 complex is dynamically coupled to the Par6-aPKC complex (Suzuki and Ono, 2006).

Tiam-1, Par3 and Par6, each one contains PDZ domains, which are by their nature bind directly to the last several, C-terminal residues of transmembrane proteins. All of the four syndecans possess the conserved II type PDZ binding site (EFYA) at the end of the cytoplasmic domain. Similar peptide sequences (EFYA/V) were isolated from a peptide library by searching with PDZ domains of Tiam-1 (Songyang et al., 1997). Indeed, every variant of syndecan-4 was pulled down in the Tiam-1 immunoprecipitation assay, however the in vivo co-localization of the syndecan-4 variants was partial.

However, anti-Tiam-1 immunostaining visualized systematically a filamentous structure in the wt- and Ser179Ala cells that structure was lost in the shRNA syndecan-4 expressing cells and a massive perinuclear accumulation was observed. In Ser179Glu the distribution of Tiam-1 was still filamentous in the cell periphery but concentrated in the perinuclear region. Interestingly the Rac1 and Tiam-1 co-localized the most in the perinuclear region in the shRNA syndecan-4 and Ser179Glu cells. On the contrary, the co-localization was less in the wt-, Ser179Ala cells, but the Rac1 and Tiam-1 distributed together. In migratory cells the Ser179Glu accumulated in the trailing edges and so did the Tiam-1, co-localizing with Ser179Glu.

We took effort to figure out which kind of a structure is decorated by anti-Tiam-1. The microtubular network was stained by α-tubuline. There was some partial co-distribution but most of the Tiam-1 filaments ran differently from the microtubular fibers. The intermedier filament cytokeratin was immunostained by pan-anti-cytokeratin antibody. Tiam-1 co-localizated much better to cytokeratin filaments than to microtubules, although anti-Tiam-1 decorated mostly the long, thick, axial cytokeratin fibers and seemingly failed to react with the cross fibers.

Taking together, presence of syndecan-4 could determine the overall Tiam-1 distributions, which highlighted some cytoskeletal structure.

Syndecan-4 and Par6 was mutually co-immunoprecipitated and *in vivo* co-localized with each other. The Par6 interaction did not differentiate among the syndecan-4 mutations nor was sensitive for GTP-GDP most probably occurred via PDZ binding.

The expression of syndecan-4 mutant lacking PDZ-binding site led to decreased cell motility and a failure to form a trailing edge (Tkachenko et al., 2006). This supports our model according to it syndecan-4 is tethered to PIP2 through its PIP2 binding motif (Couchman et al., 2002) tends to form oligomeric complexes (Tkachenko et al., 2004; Koo et al., 2006), which essential to develop a network of PDZ domain containing proteins. It participates in the Par polarity complex most probably orients it depending on its phosphorylation status. A conformational change occurs upon phosphorylation of Ser179 of syndecan-4. The PIP2 binding, and the tendency for oligomeric form is diminished (Couchman et al., 2002; Koo et al., 2006), but developing a higher affinity for α-actinin binding (Chaudhuri et al., 2005). With this syndecan-4 is leaving the TJ and AJ region and moving to the baso-lateral region. Due to the phosphorylation syndecan-4 is being capable of reducing the active Rac1 level blocking the interaction between Rac1-GDP and Tiam-1. Probably the Par6 in the baso-lateral region binds Cdc42 (Etienne-Manneville, 2004), which is implicated in several other functions such as microtubule organization. Since syndecan-4 accumulates in the trailing edges and down regulates Rac1 activity helps to form a decreasing Rac1 gradient from the lamellipodia to the trailing edge.

In a further related embodiment, the invention provides the polypeptides of the invention for use in the treatment of further specified diseases and disorders.

In another aspect, the invention provides the use of a polypeptide according to the invention for the preparation of a pharmaceutical composition suitable for enhancing the epithelial phenotype of a cell, enhancing the migratory phenotype of a cell or initiating the neuronal differentiation of a cell in a patient being in a need thereof.

The present invention is particularly suitable in assisting the treatment of cancer, especially carcinomas. Especially preferred targets are the breast adenocarcinoma, colon carcinoma, gastric carcinoma, prostate carcinoma, endometrial carcinoma, and melanoma. By modulating the activity of Rac1 and therefore influencing the migratory phenotype of tumor cells, the polypeptides and methods of the invention will allow the inhibition of cells becoming metastasized or the process may be advantageously reversed. The limitation or elimination of metastasis enhances the chance for operating successfully the tumors, or applying other therapies with more chance of success.

In addition, the present invention may further be useful in other diseases and disorders, in particular and without limitation in neuropsychiatric disorders including schizophrenia, depression, Alzheimer's disease, eplilepsy, HIV encephalitis, neurodevelopmental disorders including nonspecific X-linked mental retardation, fragile X mental retardation syndrome, tuberous sclerosis complex, autism, Rett syndrome, Down syndrome, Wiskott-Aldrich syndrome, Chronic granulomatous disease (CGD), leukemia, angioproliferative disease, ischemia or excitotoxicity, traumatic nerve injury, polyglutamine diseases, sympathetic neuron death, amyotrophic lateral sclerosis, wound healing with keratinocytes and fibroblasts migration: venous and diabetic ulcers, muscle-regeneration with satellite cells migration, myoblast fusion.

With respect to the neuronal differentiation, the present invention provides specific advantages, wherein the process is shown to be dependent on syndecan-4 phosphorylation. Further, the syndecan-4 can initiate the neuronal differentiation of carcinoma cell lines, rather than being a neural marker thereof.

In further embodiments of the present invention, the polypeptide is of synthetic origin with well defined properties in achieving the aim of the invention. In particular, polypeptides having the sequence of SEQ.ID.NO.: 1 or SEQ.ID.NO.: 2 are suitable for the up-regulation of Rac1 activity. The polypeptides having the sequence of SEQ.ID.NO.: 3 or SEQ.ID.NO.: 4 are suitable for the down-regulation of Rac1 activity.

In another aspect a method is disclosed for identifying an agent capable of indirectly modulating the activity of the small GTPase Rac1 comprising the steps of:
a) contacting an agent of interest with a cell comprising syndecan-4 molecules with a known ratio of phosphorylated and de-phosphorylated serine residue at position 179 (Ser179);
b) determining whether said phosphorylation ratio in said cell is significantly changed after said contacting;
c) identifying said agent as an agent capable of modulating the activity of Rac1 if said phosphorylation ratio in said cell is significantly changed after said contacting.

Syndecan-4 is expressed ubiquitously, so it is present on virtually all cell types. Therefore, the person skilled in the art usually does not have to perform any extra steps to determine whether the cell of interest expresses syndecan-4 on its surface. However, this measurement may help the person skilled in the art in the optimization of the steps of the method according to the invention, or the determination may show that the level of syndecan-4 on the cell surface is appropriate to the intended screening protocol. Conversely, where the level of syndecan-4 expressed on the cell surface is too low for the intended application, the person skilled in the art may introduce exogenous syndecan-4 into the cell by using any well-known protocol in the art. Specific examples of particularly suitable cells are: HEK293 cells, MCF7 cells, MDCK cells or HT1080 cells.

The cell of interest may be present in any type of sample. In particular, the cells may be in a cell or tissue culture, in a biopsy sample, in an intact organ, or may be isolated cells, or the step of contacting the cells and syndecan-4 may be performed in vivo.

The agent capable of indirectly modulating the activity of the small GTPase Rac 1 through interaction with syndecan-4 is not limited in any way to any class of biomolecules. Therefore, the agent may be a polypeptide, polysaccharide, nucleic acid or small molecules. To ascertain whether any given molecule is able to indirectly modulate the activity of Rac1, the person skilled in the art can perform any appropriate state of the art protocol designed to measure the activity of Rac1 without undue experimentation. The agent further can be any molecule present in the nature within the living organisms, or it may be an artificial molecule designed to have a specific effect on the organism.

The ratio of phosphorylated and non-phosphorylated serine residue should be known for carrying out the method according to the invention. However, the term "known ratio" does not necessarily refers to an absolute value or number, rather only signifies that the state of the cells used in the method of the invention should be reproducibly controllable by the person skilled in the art throughout the steps or different parallel samples. Accordingly, the change of this ratio is what needs to be monitored, and the protocol used should be able to detect this change in the phosphorylation state of Ser179.

In another approach, the ratio of phosphorylated serine residue may be given in terms of the percentage of the total syndecan-4 Ser179. For example, the amount of phosphorylated Ser179 of syndecan-4 may be about 10% of the total available Ser179 in a given cell, or any other physiologically relevant value, such as 1%, 2%, 5%, 10% 15%, 20%, 25%, 30%, 40%, 50% or above that. In addition, the person skilled in the art will appreciate that the amount and/or ratio of the Ser179 of syndecan-4 may vary depending on many factors, i.e. based on the cell type selected, the developmental state of the cell, stress, cell cycle, etc. Accordingly, for the routine practicing of the present method the person skilled in the art needs to take these factors into consideration and standardize the conditions to be able to get reliable results.

In any case, the person skilled in the art is capable to select an appropriate protocol for determining the phosphorylation state of said Ser179; it can be any protocol available to the person skilled in the art suitable to measuring the amount of phosphorylation on the amino acid residue in the Rac1 polypeptide. These protocols are known to the person skilled in the art, for example standard immunological detection may be performed with specific antibodies against the phosphorylated and non-phosphorylated serine residue with usual ELISA or Western blot protocols, or with immunohistochemistry.

The length of the contacting step may differ in different applications. However, the person skilled in the art will be able to determine the time necessary by using routine optimization without undue experimentation. Typical time to allow the interaction can be as short as a few minutes, or it may be as long as a few hours, preferably between 10 minutes and 3 hours, more preferably between 30 minutes and 2 hours, or about 1 hour.

After the contacting step, the ratio of phosphorylated and non-phosphorylated Ser179 is determined, according the protocol mentioned above, and the change in the ratio is calculated. The change is "significant" if the ratio is shifted at least 10%, preferably 30%, even 50% or more.

Finally, in the last step of the method according to the invention, the agent is identified as an agent capable of modulating the activity of Rac1 if said phosphorylation ratio in said cell is significantly changed after said contacting.

### Brief description of the figures

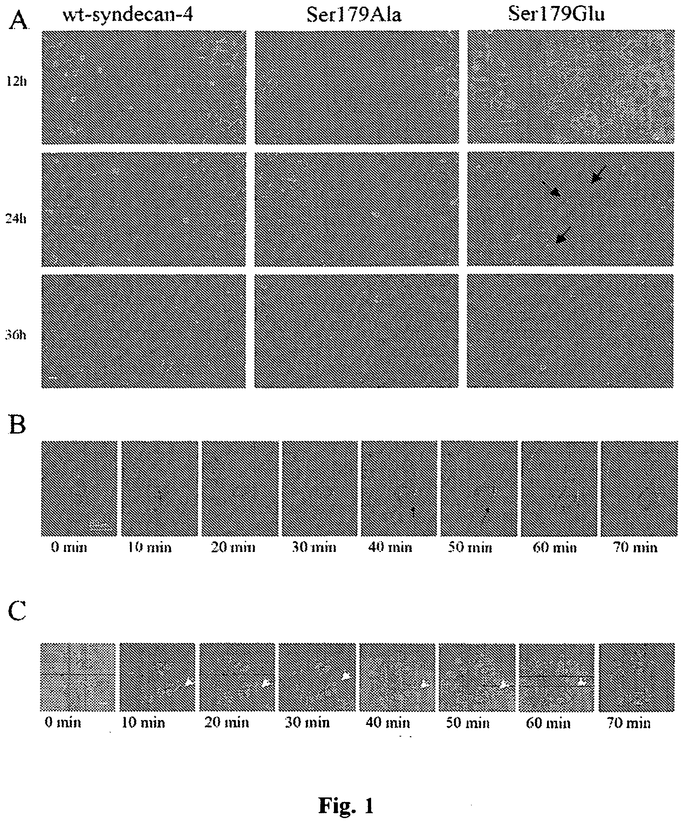

Fig. 1. **Ser179 mutation induced migration in MCF-7 cells. (A)** Scratch-wound assay was performed with stable transfected MCF-7 lines expressing wild type, and Ser179Ala or Ser179Glu mutant syndecan-4 at 80% confluency. The photos were taken 12 h, 24 h and 36 h after scratch wounding. Arrows mark the cells with migratory morphology in the wounded area. **(B)** Untreated Ser179Glu-expressing cells migrate individually on fibronectin. The arrows show lamellipodia formation. (C) A cluster of Ser179Ala-expressing MCF-7 cells migrates collectively upon 1 µM treatment of PMA. White arrows point to the rear cell that made the cluster move ahead forming trailing edges while the lateral cells were heavily ruffling. Time-lapse photos were taken in every ten minutes in B and C. The glass coverslips were covered with 10 µg/ml fibronectin. Bar, 50 µm in A, B, C.

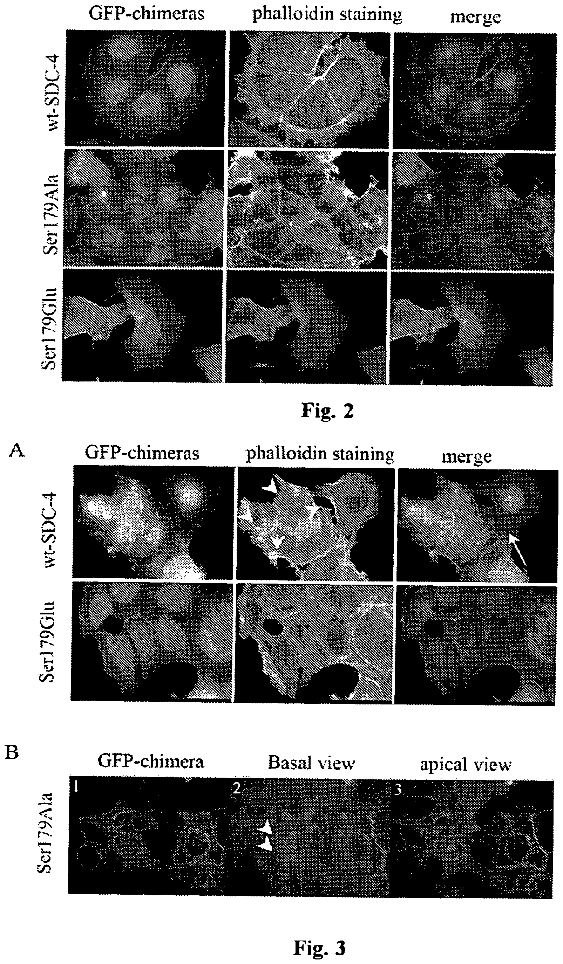

Fig. 2. **Organization of the actin cytoskeleton in the cell lines expressing wild type or mutant syndecan-4.** MCF-7 cells expressing the different syndecan-4-GFP chimeras were fixed, permeabilized and stained with TRITC-conjugated phalloidin (red), syndecan-4-GFP chimeras were visualized by green fluorescence. Wild type and Ser179Ala syndecan-4 accumulated in the perinuclear region and weakly co-localized with the cortical actin bundles. No stress fibers were seen but adherens actin belt were dominant. The Ser179Glu syndecan-4 co-distributed along the stress fibers. Nuclei were stained by Hoechst (blue). Bar, 20 µm.
Fig. 3. **Phorbol ester (PMA) changes the actin cytoskeleton.** MCF-7 cells expressing the different syndecan-4 variants were incubated with 1 µM PMA for 60 min and actin was visualized by phalloidin (red). Administration of PMA resulted in the reorganization of stress fibers in wt- and Ser179Ala cells (A; B2) pointed by white arrowheads. Solitary cell migration was stimulated in wt-syndecan-4 shown in the panel A, where a white arrow pointed at a cell leaving the cluster with typical actin cytoskeleton of migratory phenotype. On the contrary, PMA incubation of Ser179Glu cells changed the migratory phenotype to quiescent phenotype relaxing the stress fibers to loose concentrical actin rings. On the panel B Ser179Ala syndecan-4 (green) highlighted the plasma membrane (B1). Upon PMA stimulation stress fibers were reorganized at the basal surface (B2) whereas the adherens actin belts were unchanged in the apical region (B3) visualized in different focal planes. Nuclei were stained by Hoechst dye (blue). Bar, 20 µm.

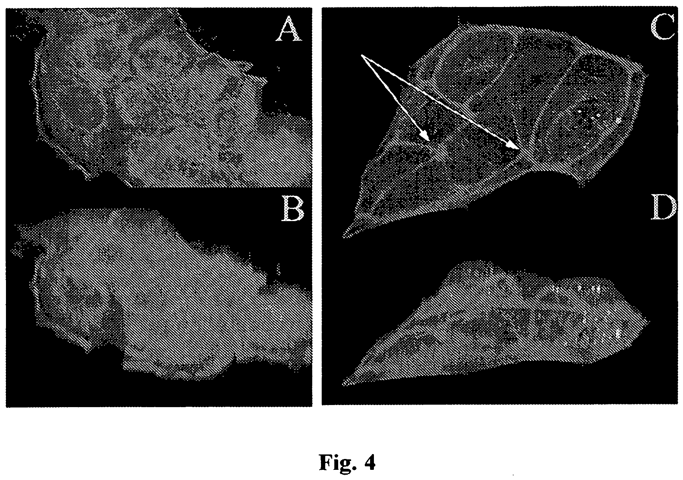

Fig. 4. **Actin structures of clusters of wt- and Ser179Ala.** Cell clusters expressing wt-syndecan-4-GFP chimera (green, A, B) or Ser179Ala syndecan-4 (green, C, D) were stained with phalloidin (red) and are shown from top (A, C) or from side (B, D). Thick, continuous circumferential actin cables were organized around the Ser179Ala cluster (C), which is in not intact around the wt-syndecan-4 cluster. In the Ser179Ala syndecan-4 cells junctional actin bundles aligned with the cell borders shown in panel C, which formed meshwork of arches connected the peripheral actin cortex (D). At centers, where more than two cells met the actin arches organize nodes pointed by white arrows, and a massive nucleation can be seen (C). The Ser179Ala syndecan-4 formed small vacuoles co-localizing with the actin arches. The height of the middle cells of Ser179Ala line is 30 µm while the average height of the wt-syndecan-4 is 20 µm.

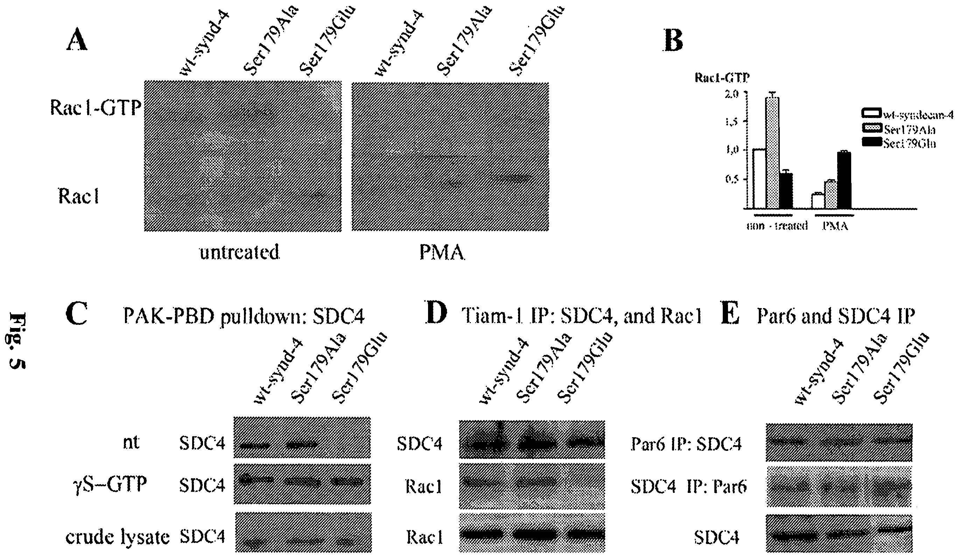

Fig. 5. **The level of Rac1-GTP is influenced by the expression of syndecan-4 constructs.** Activation of Rac1 (Rac1-GTP) was monitored in cells expressing wild type or Ser179Ala, Ser179Glu mutant syndecan-4 (panel A). In the upper slab the amount of active Rac1 (Rac1-GTP) is shown, in the lower slab total Rac1 is represented as equivalent loading between experiments. Among the cell lines the Ser179Ala syndecan-4-expressing cells contained the most Rac1-GTP and Ser179Glu expressions contained the least activated Rac1. Upon PMA stimulation the amount of Rac1-GTP of the different cell lines were changed; decreased in the wt-and Ser179Ala syndecan-4 expressing cells and increased in Ser179Glu cells. Signal intensities were quantitated and drawn in the diagram of panel B.

The filter of the Rac assay was probed with anti-syndecan-4 antiserum (panel C). The samples of wt-syndecan-4, Ser179Ala, Ser179Glu, respectively, were assayed with γ-S-GTP according to the manufactures's suggestions to even the concentration of Rac1-GTP, lanes of 4-6. The amount of the pulled-down syndecan-4 was similarly changed to that of Rac1-GTP clone by clone (lane 1-3), however, the equalized Rac1-GTP level diminished the difference among the syndecan-4 clones pulled down (lane 4-6). The bottom lanes (7-9) represent the equal loading.

Immunoprecipitation with Tiam-1 antiserum was tested with anti-syndecan-4, or anti-Rac1 antisera (panel D), in the lanes 1-3, and lanes 4-6, respectively. There was no difference in the amount of the pulled down syndecan-4 variants detected on the filter (lanes 1-3). In the wt- and Ser179Ala cases the signals of Rac1 (lanes 4-6) were similar, however it was greatly reduced in the case of Ser179Glu expressing cells although the different variants contained equal Rac1 level presented in the lanes 7-9.

Syndecan-4 and Par6 co-immunoprecipitated each other (panel E). In the immunoprecipitate of anti-Par6 antiserum syndecan-4 or in the immunoprecipitate of syndecan-4 Par6 were observed. The syndecan-4 mutations did not alter the amount of the detected proteins (lanes 1-6). The equal loading was represented by the lanes 7-9.

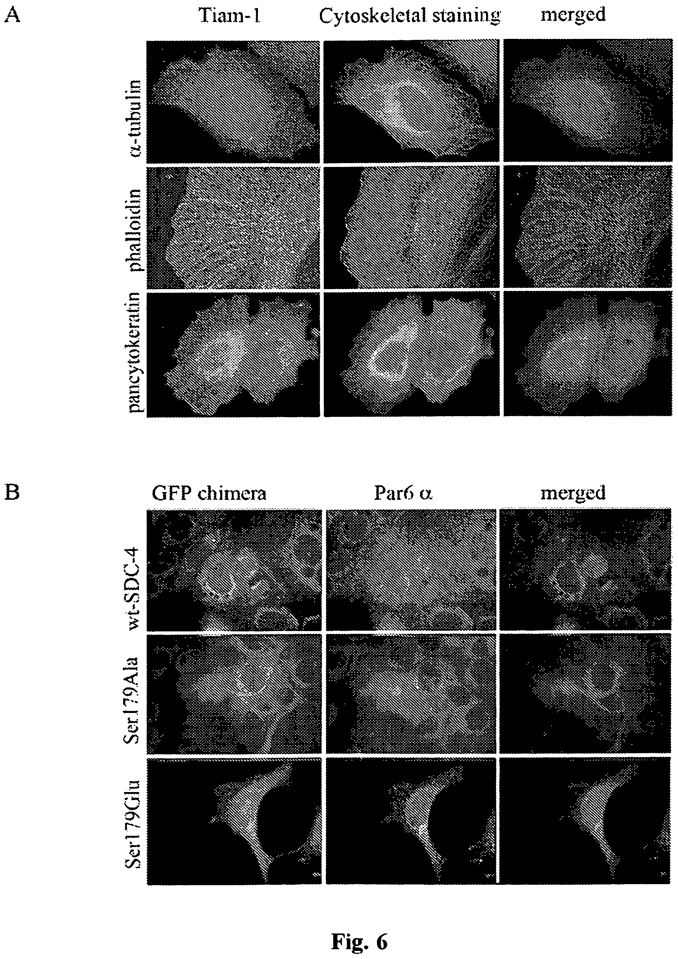

Fig. 6. **Distributions of Tiam1 and Par6 are mediated by syndecan-4 in a phosphorylation dependent manner.** In the panel A Tiam-1 (green) was visualized by anti-Tiam1 antiserum (SC-872) revealed a fibrillar structure. Double staining was applied to identify the nature of the fibrils with different cytoskeletal factors (red) in wt-syndecan-4 expressing cells. Tiam1 avoided the co-localization with microtubules or with actin immunostained by anti-α-tubulin and phalloidin, respectively. However, co-distribution was observed with cytokeratin in the perinuclear region visualized by pan-anti-cytokeratin. Par6a (red) and syndecan-4 - GFP chimeras (green) were monitored in different syndecan-4 variants expressing lines in panel B. Par6 was co-distributed with the studied syndecan-4 variants: wt-syndecan-4 and Ser179Glu that means that similarly to Ser179Glu Par6 concentrated in the trailing edges in migratory cells.

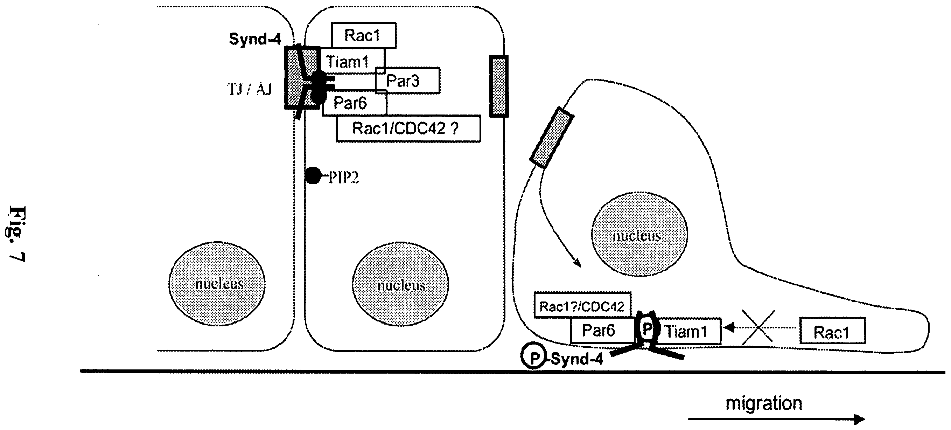

Fig. 7. **Syndecan - 4 mediates the localization of Par polarity complex in a phosphorylation dependent manner.** Schematic representation of syndecan-4 activities according to the invention.

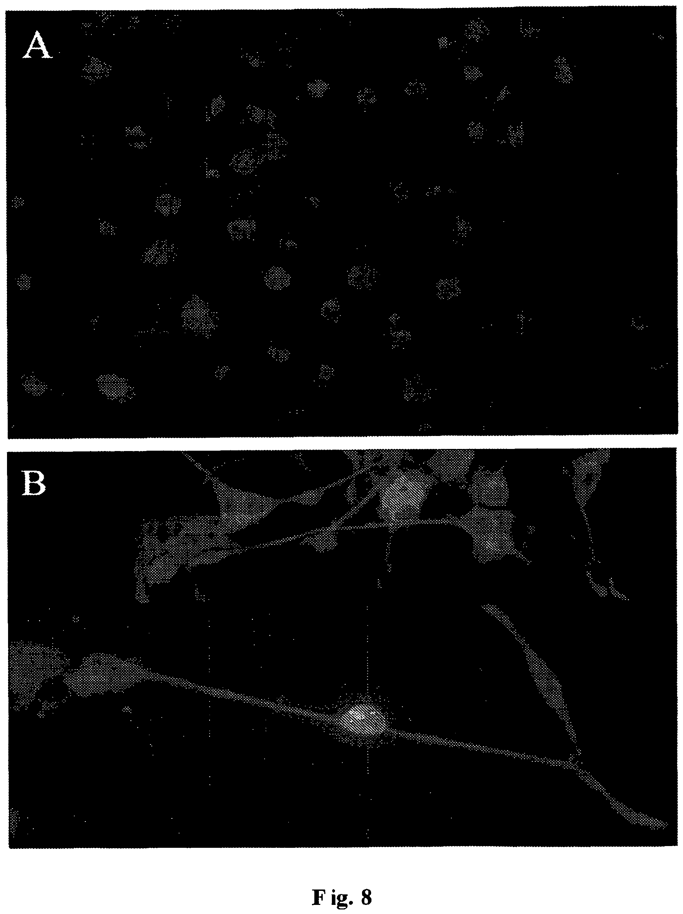

Fig. 8. **Neuronal differentiation induced by short Syndecan-4 peptides.** MCF7 cells were transfected with wild type syndecan-4 (A) and DEGEpC3 (B), respectively. Expression of DEGEpC3 (green) resulted in neuron - like phenotype upon serum starvation. Red : b3-tubulin staining. Blue: Hoechst nuclear staining (A). DEGEpC3: MYRMKKKDEGEYDL was joined to GFP.

The present invention is further illustrated by the experimental examples described below, however, the scope of the invention will by no means be limited to the specific embodiments described in the examples.

### Example 1: Materials and methods

### Cell culture

The MCF-7 breast adenocarcinoma cells were obtained from ATCC, cultured in DMEM:F12 (Cambrex, Walkersville, MD, USA) supplemented with 10% fetal bovine serum (FBS) (Gibco, Bethesda, MD, USA), otherwise stated. The stably transfected cell lines were propagated in DMEM:F12 medium supplemented with 10% FBS and 200 µg/ml G418 (Cambrex, Walkersville, MD, USA). The cell lines were seeded onto glass coverslips or plastic dishes coated for 2h with 10 µg/ml fibronectin (Sigma-Aldrich, St. Louis, MO, USA).Scratch-wound assays were performed on stable transfected Syndecan-4 wild, Ser179Ala and Ser179Glu cells at 80% confluency. The cultures were scratched with a sterile P10 pipette tip 3-4 times in parallel lines. Images were taken 12, 24, 36 and 48h following wounding.

In the cell migration assay 60% confluent MCF7 cells or the stably transfected lines were stimulated by 1 µM phorbol-12-myristate-13-acetate (PMA; from Sigma-Aldrich St. Louis, Mo, USA), otherwise stated. The images were taken using 10x, 20x0.3 objective at 3-min intervals for 2h using inverted microscope (Leica) and organized into time-lapse movies in Adobe ImageReady® v.8.

### cDNA constructs and transfection

The human syndecan-4 was isolated and cloned into pCMV vector (Clontech, Heidelberg, Germany). The green fluorescent protein (GFP) was inserted behind the cell binding domain (CBD) of the extracellular domain (McFall and Rapraeger, 1997) into the position of 131 of the syndecan-4 amino acid sequence. The Ser179 syndecan-4 was mutated to Ala or Glu obtaining Ser179Ala or Ser179Glu syndecan-4, respectively. The MCF7 cells were transfected with the plasmids of interest by using FuGene6 transfection reagent (Roche Diagnostics, Mannheim, Germany) according to the manufactttrer's instructions. For stable expression the transfected cells were selected in medium supplemented with 500 µg/ml G418 (Cambrex).

Mouse monoclonal anti-vinculin, anti-vimentin antibodies (Sigma-Aldrich), mouse monoclonal anti-focal adhesion kinase (clone 4.47, Upstate Biotechnology Inc., Lake Placid, NY, USA), mouse monoclonal anti-paxillin antibody (clone 5H11, Thermo Fisher Scientific, Labvision Cheshire, UK), mouse monoclonal anti-alpha actinin antibody (Chemicon, Millipore, Billerica, MA,USA), rabbit polyclonal anti - coflin, anti-phosphocofilin antisera (Cell Signaling, Danvers, MA, USA), mouse monoclonal anti-GFP antibody and rabbit polyclonal antibody raised against the synedcan-4 ectodomain (Invitrogen/Zymed Corporation, Carlsbad, California, USA) were used according to the manufacturer's instruction. F-actin was visualized with TRIC-conjugated phalloidin (Sigma-Aldrich). Hoechst 33258 (Sigma-Aldrich) staining was used to label nuclei.

For immunofluorescence, the primary antibody was detected using Cy3-conjugated goat anti-mouse IgG andCy2-conjugated donkey anti mouse F(ab'), F(ab')2 fragment antibodies were purchased from Jackson ImmunoResearch Laboratories (West Grove, PA, USA ). HRP-conjugated anti mouse and anti-rabbit secondary antibodies were purchased from DAKO (Glostrup, Denmark).

### Immunofluorescence microscopylassay

Syndecan-4 wild type, Ser179Ala and Ser179Glu transfected cells were seeded on fibronectin coated glass coverslips. The cells were incubated with 1 µm PMA for 1 h in the normal growth medium, otherwise stated.

For immunocytochemistry the cells were fixed for 5 min with 4% paraformaldehyde after washing at room temperature and permeabilized with 0.1% Tween20 for 5 min. The cells were incubated for 30 min at room temperature with primary antibodies diluted in 2% BSA according to the manufacturer's suggestions, then were washed 3 times with phosphate-buffered saline (PBS), incubated with secondary antibodies in 2% BSA for 30 min at room temperature. After Hoechst nuclear staining the coverslips were mounted with Fluorescent Mounting Medium (DAKO). Cells were studied with Zeiss Axio Imager (Carl Zeiss, Jena, Germany), Nikon6000 microscope, or BioRad MRC-1024 (Richmond, Ca, USA) confocal microscope. Images were analysed using Adobe Photoshop® v.8.

### Racl GTPase pulldown assay

Rac1 activity was characterised by applying RAC-activation kit purchased from Millipore (Upstate) and it was used according to the manufacturer's prescription. Briefly, p21-binding domain of PAK-1 was fused to agarose beads, which specifically binds to and precipitates Rac-GTP and Cdc42-GTP from cell lysates. Cells were harvested at 70% confluency of 25 ml flasks. The wild type Syndecan-4, Ser179Ala and Ser179Glu cells were treated for 60 minutes with 1 µM PMA (Sigma-Aldrich), or 500nM rottlerin (Sigma-Aldrich), or 10 nM Gö6976 (Biosource, Camarillo, CA, USA). The active Rac1 was detected by Western blotting using mouse anti-Rac1 antibody (Upstate). To test whether GST-Pakl-Rac/Cdc42-binding domain could pull syndecan-4 down, the blot was probed with rabbit polyclonal anti-syndecan-4 or mouse monoclonal anti - GFP antibody.

### Immunoprecipitation, pull-down assay

Immobilized protein A/G was purchased from Pierce (Rockford, IL, USA). Immunoprecipitation was performed according to supplier's suggestion. In brief, 70% confluent cultures of 25 mL flask were detached, pelleted and following 2 times washing with PBS. The pellet were lysed in RIPA buffer then centrifuged and the supernatant was pre-cleared by addition of 5 µL of protein A/G-sepbarose beads slurry (Pierce) for 2 hours, then the supernatant was incubated with the antibody of interest for 24 hours. 10 µL of protein A/G slurry was added for 2 hours, collected by pulse centrifugation, washed 3 times with lysis buffer, resuspended in 2X SDS loading buffer subjected to SDS-PAGE, followed by immunoblotting with the appropriate antibodies.

### Example 2: Scratch-wound assay implies migratory phenotype of Ser179Glu

The wt-syndecan-4, Ser179Ala, and Ser179Glu lines were tested how they close the scratched wound on fibronectin substratum. At 80 % confluence the cultures were scratch-wounded and healing was monitored. Still images were captured at the indicated time points (Fig. 1). In monolayers of the wt-syndecan-4 and the Ser179Ala lines the wounds healed from the edges, i.e. progenies of cell division narrowed the scratch but kept tight contact with the cell layer. However, some cells of the Ser179Glu line appeared in the center of the scratched area by 12 hours following wounding indicating enhanced cell motility (Fig. 1A). The gaps closed in every case almost at the same time, in 42 hour. There was no difference in the growth rate of the different lines measured by SRB assay (data not shown).

The non-transfected (nt) MCF-7 and the cells expressing the wild-type and the Ser179Ala syndecan-4 exhibited epithelial morphology. The cells abutted each other in a uniform array, formed a cobblestone pattern and held themselves tightly together building on clusters. The Ser179Glu expressing cells, however, exhibited a less uniform organization and scattered evenly on the flask until 60 % of confluence. The individual Ser179Glu cells frequently formed lamellipodia, filopodia and trailing edges characteristic for migrating cells.

### Example 3: The phospho-mimetic Ser179Glu mutation is sufficient to trigger migration whereas the amount of vinculin is reduced in the focal adhesions

Results of the previous scratch - wound assay gave a hint that the Ser179Glu cells gained a migratory phenotype. To observe cell migration the Ser179Glu-expressing cells were plated on fibronectin-coated glass coverslips and still images were captured in every 3 minutes. Selected frames focusing on a migrating cell are shown in Fig. 1B. The average migration velocity of the cells was 0.4 ± 0.1 µm/min.

To unravel the changes in the cytological architecture that gave basis of the migratory phenotype immunocytochemical investigation was carried out with phalloidin, anti-FAK, and anti-vinculin antisera. The cells of nt-MCF-7, and wt-syndecan-4 lines did not exhibit cell motility on fibronectin-coated surface without proper stimulation. Phalloidin staining of cytoskeleton visualized cortical actin rings and there were no stress fibers detected (Fig. 2). The cells were well spread and attached to the fibronectin-coated bottom via evenly distributed mature focal adhesions that were approximately 60 per cell, visualized by anti-FAK or anti-vinculin antibodies (Fig. 2). Syndecan-4 was not or hardly detected in focal adhesions of quiescent cells.

The individual cells of the Ser179Ala line were well spread but in the clusters the neighboring cells compacted towards each other elevating the height of the cells, and no migration was observed. No mature focal adhesions were detected by either anti-vinculin or anti-FAK antisera. Only small focal complexes were seen, in which Ser179Ala syndecan-4 was partially observed (Fig. 2). The Ser179Ala syndecan-4 was distributed along the actin filaments, accumulated in the plasma membrane and was also found in filopodia.

Phalloidin staining of the Ser179Glu line revealed that without any treatment the typical cortical actin filaments were rearranged to stress fibers, which is characteristic for migratory cells. The Ser179Glu mutant syndecan-4 was accumulated in the trailing edges co-distributed abundantly along the stress fibers and concentrated at the tip of the stress fibers (Fig. 2). Immunostaining of FAK visualized well-developed focal adhesions, but immunoassay with anti-vinculin failed to stain any of those, although the cytoplasm showed strong vinculin accumulation (Fig. 2).

### Example 4: PMA activation has opposite effect on the quiescent or the migratory cells

PMA (phorbol-12-myristate-13-acetate) induction was previously shown to stimulate cell migration of nt-MCF-7 cells (Johnson et al., 1999). Our different cell lines were also challenged with PMA. In the case of the nt-MCF-7, wt-syndecan-4 expressing cells the cortical actin filaments rearranged into stress fibers at the rear and dorsal actin fibers appeared in the front. The GFP - tagged wt-syndecan-4 distributed along the stress fibers but was hardly seen with the actin arcs. The size of focal adhesions was reduced at the leading edge to focal complexes and increased at the trailing edge (Fig. 3). After 10 min PMA stimulation solitary cells detached from the clusters and started migrating with the velocity of 0.3 ± 0.1 µm/min at 25 °C (Supplement).

However, when the Ser179Glu cells were incubated with 1 µM PMA, surprisingly, cell-migration stopped and the cells became more spread with dominant epithelial morphology. The stress fibers relaxed and reorganized to cortical actin filaments (Fig. 3). In 30 min PMA incubation vinculin started appearing in the focal contacts and 24 hours of PMA treatment resulted in a formation of well-developed focal adhesions, which frequently appeared concentrically around the nucleus. Ser179Glu syndecan-4 was hardly found co-localized with vinculin containing focal adhesions. Interestingly, anti-FAK antibody could visualize regularly distributed and sized focal adhesions (Fig. 3) co-localizing with Ser179Glu syndecan-4 in the PMA treated cells, too.

Taking together, upon PMA incubation the basically quiescent cells of the nt-MCF-7 and wt-syndecan-4 lines gained migratory ability; but the migratory cells of the Ser179Glu line stopped migration, the stress fibers reorganized to cortical actin rings and vinculin reappeared in the focal adhesions.

### Example 5: PMA induced collective migration in the Ser179Ala lines

The cells of the Ser179Ala line were also monitored upon PMA stimulation. Still images were captured and the video analysis revealed that following PMA incubation the tightly adhered Ser179Ala cells moved collectively as a sheet, contrary to the individually migrating cells of nt-MCF-7 or wt-syndecan-4 lines on fibronectin-coated surface. The peripheral cells formed the leading edge utilizing actin-mediated ruffles. The clusters were usually moved by one motor cells at the rear, in which stress fibers were developed (Fig. 4). If an individual migratory Ser179Ala expressing cell met a cluster, united to the sheet and forced the whole group to move with similar rate but the direction was dependent on the other cells in the clusters showing an enhanced competence for cell adhesion. The clusters were moving with similar rate, approximately 0.3 µm/min, as the individual cells of the other lines. Collective migration was not observed in the nt- MCF-7 cells or those expressing wt-syndecan-4. The small focal complexes developed to mature focal adhesions stained by anti-vinculin and anti-FAK antisera (data not shown).

### Example 6: Inhibition of PKCα activity promotes motility of wt-, and Ser179Ala cells

Previously published data supported that syndecan-4 determinates the activity of PKCa (Koo et al., 2006, Chaudhuri et al., 2005), where P-Ser179 syndecan-4 decreases PKCa activity (Murakami et al., 2002). Introduction of the phospho-mimetic Glu into the position of 179 was a gain-of-function mutation resulting in improved migration. The question was raised whether it was due to the effect of down regulation of PKC activity. To unravel the potential role of PKCα G66976, a potent PKCα specific inhibitor was applied to the different lines.

Incubation with 10 nM G66976 had diverse effects on the cell lines tested. Vinculin could be hardly observed in the focal adhesions in the treated cells of the nt-MCF7 and wt-syndecan-4 line (data not shown). However, Ser179Ala cells stimulated with G66976 developed strong, mature focal adhesions visualized by anti-FAK and anti-vinculin antisera (Fig. 4). Strong ruffling was observed in the Ser179Ala cells and moderate in the wt-syndecan-4 cells. Individual cells and clusters migrated with a very low velocity of approximately 0.05 ± 0.02 µm/min in the wt-syndecan-4 and Ser179Ala lines.

Taking together, the applied PKCa inhibitor affected the composition of focal adhesions, improved maturation of the focal complexes to focal adhesions in the Ser179Ala line, and was able to induce cell migration in the same cell line. It means that PKCα has important but not essential role in cell migration.

### Example 7: Syndecan-4 affected the organization of actin bundles

The individual Ser179Glu expressing cells showed migratory phenotype and reorganized actin cytoskeleton to stress fibers that were seen in the cells of nt-MCF-7, and wt-syndecan-4 lines upon PMA stimulation only.

The cells expressing Ser179Ala syndecan-4 showed enhanced epithelial morphology. Even in low density of cells clusters were formed where the neighboring cells were compacted towards each other resulting in taller cells, which was also seen in wt-syndecan-4 expressing cells in higher density of cells in the flasks. We could distinguish several organization levels of actin filaments visualized by phalloidin staining the clusters during epithelial polarization. (1) At the bottom of the peripheral cells sometimes short stress fibers appeared upon proper stimulation (Fig. 2, Fig. 3, Fig. 4, Fig. 5). (2) On the basal surface, without any stimulation loose band of concentrical cortical actin rings were detected (Fig. 5) and (3) around the clusters thick actin cables were seen (Fig. 4). Detailed Z stack analysis revealed that these thick actin belts around the cluster of epithelial wt-syndecan-4 and Ser179Ala cells ran not exactly on the basal but on the lateral surface close to the basal surface and constituted from thin actin fibers (Fig. 2, Fig. 3, Fig. 5), further (4) on the lateral sides of wt-syndecan-4 cells in the early stage of polarization vertical actin rods were observed (Fig. 5). During the epithelial polarization in Ser179Ala cell line the actin rods disappeared and (5) junctional actin rings were organized around each cells of a sheet in the adhesion belt, aligned with the cell borders (Fig. 3, Fig. 4, Fig. 5), which, indeed were constituted from actin arches shown in the side view (Fig. 5).

Prior to the epithelial polarization the average height of the quiescent cells were 18±4 µm contrary to the polarized epithelial cells where the average height was increased up to 33±3 µm and the cells became more cuboidal. The junctional actin bundles organized in the adherens junction, at the apical pole. Because of the geometry of a small cluster these junctional actin bundles appeared as actin arches, which were cross-connected to each other constituting a meshwork and tethered to the basal actin cables at the peripheral cells. This structure was similar to the dome tent, most probably with similar support function (Fig. 5). Phalloidin highlighted some central nucleation points at the interception of several arches wherein Ser179Ala syndecan-4 was observed shown as yellow spots (Fig. 5). The junctional actin bundles were also observed in nt-MCF-7, wt-syndecan-4 expressing cells above 80 % confluence but rarely in the case of Ser179Glu.

Taking together, non-transfected, wt-syndecan-4 and Ser179Ala MCF-7 cells formed clusters. The Ser179Ala mutation enhanced the epithelial phenotype and initiated the polarization of epithelial cells.

### Example 8: The amount of Racl-GTP is modulated by syndecan-4

Previous studies supported that syndecan-4-null cells did not develop mature focal adhesions (Ishiguro et al., 2000; Bass et al., 2002). In syndecan-4 knockout cells elevated Raci level was detected with small focal complexes (Saoncella et al., 2004; Tkachenko et al., 2004; Bass et al., 2002), in accordance with the classical observation of Nobes and Hall (1995) that increased active Rac1 concentration resulted in small focal complexes. Since in the Ser179Ala-expressing cells only small focal complexes developed, which may have indicated a higher level of activated Rac1, thus we compared the level of Rac1-GTP in the different cell lines. Indeed, the expression of Ser179Ala elevated the level of activated Rac1 (Fig. 6) up to 1.9 ± 0.15-fold in comparison with the wt-syndecan-4 line. On the contrary the expression of Ser179Glu syndecan-4 decreased the relative concentration of Rac-GTP to 0.6 ± 0.1 compared to wt-syndecan-4. Previous studies indicated that PKC-s could influence the level of the activated Rac 1 (Bass et al., 2002). Thus we estimated the RAC-GTP level upon PKC activation with PMA, and specific inhibition of PKCα with Gö6976. Upon PMA stimulation the Rac1 activity decreased to 0.25 and 0.4 of the non-treated wt-syndecan-4 in the wt- and Ser179Ala syndecan-4 cells compared to the non-treated values, respectively, however, the level of Rac-GTP increased in the Ser179Glu cells almost to the normal value of 0.9 ± 0.2 of the non-treated wt-syndecan-4. Administration of the PKCα inhibitor Gö6976 reduced the activated Rac1 level in all three cell-lines. In the case of Ser179Ala cells the concentration of GTP-Rac1 was decreased to the level of wt-syndecan-4 (1 ± 0.1) but it was greatly reduced in the cases of wt- and Ser179Glu syndecan-4 lines, 4.2 ± 0.4-fold and 16 ± 2-fold, respectively. (Fig. 5; Table 1).

**Table 1: Measured values of stimulation the Rac1 activity and migration.**

| | migration (µm/min) | | | relative Rac 1-GTP level | | |
|---|---|---|---|---|---|---|
| treatment | nt | PMA | Gö6976 | nt | PMA | Gö6976 |
| wt-syndecan-4 | n/a | 0.3 ± 0.1 | 0.05 ± 0.02 | 1 | 0.25 ± 0.05 | 0.25 ± 0.03 |
| Ser179Ala | n/a | 0.3 ± 0.1 | 0.05 ± 0.02 | 1.9 ± 0.15 | 0.46 ± 0.05 | 1 ± 0.01 |
| Ser179Glu | 0.4 ± 0.1 | n/a | n/a | 0.6 ± 0.1 | 0.95 ± 0.06 | 0.06 ± 0.02 |

Taking together, the level of GTP-Rac1 was determined by the syndecan-4 construct; the highest Rac1-GTP was observed in the Ser179Ala line, where enhanced epithelial polarization was detected. The lowest level of active Rac 1 was in the migratory Ser179Glu expressing cells.

### Example 9: Syndecan-4 can be pulled down by GTP loaded p21-binding domain of PAK, and co-immunoprecipitated with Tiam1

We tested whether the protein complex pulled down with the Rac1/CD 42-binding domain of PAK (PAK-PKB) used in the Rac1 assay contained syndecan-4. Parallel filter of the Rac1 assay was probed with anti-syndecan-4 or anti-GFP antisera. The syndecan-4-GFP monomers migrating with 55 kDa relative electrophoretic mobility gave strong signals (Fig. 6). The amount of pulled-down syndecan-4 was in accordance with Rac1 level detected in the different syndecan-4 constructs (Fig. 6). The most syndecan-4 was pulled-down from the Ser179Ala line, approximately 20 % less from the wt-syndecan-4 line, and there was only hardly detectable amount in Se179Glu syndecan-4 cells (Fig. 6). When γ-S GTP was applied to the cell lysates to equalize the pulled-down Rac1-GTP level there was no difference in the amount of pulled-down syndecan-4 variants (Fig. 6). So, the affinity of the different syndecan-4 mutants is the same to the GTP-Rac/Cdc42-PAK complex. The GDP loaded sample that is considered as negative control could not pull down active Rac1 and syndecan-4 either (data not shown).

Since Tiam-1 is a Rac1-GEF (GTP exchange factor) and it could be pulled down by PAK-PKB (Mendoza-Naranjo et al., 2006) we tested whether syndecan-4 can be co-immunoprecipitated by anti-Tiam-1. The syndecan-4 chimeras were detected by anti-syndecan-4 and anti-GFP antisera at 55 kDa and 90 kDa length, which were correspondent to the monomer and dimmer forms, respectively. Because the co-sedimentation of syndecan-4 by PAK-PKB was GDP-GTP dependent we tested if the GDP or GTP had effect for the amount of the anti-Tiam-1- co-immunoprecipitated syndecan-4 forms. From the cell lysate saturated by GDP the Ser179Ala and wt-syndecan-4 were dominant contrary to the γ-S GTP saturated case, where the Ser179Glu syndecan-4 was detected abundantly.

Taking together, syndecan-4 could be pulled down by the activated Rac/Cdc42 assay. The amount of the pulled-down syndecan-4 was dependent on the GTP-Rac1 level but the mutation of Ser179 had no effect on the binding of syndecan-4.

### Example 10: Syndecan-4 interacts with the components of the Par polarity complex: Tiam-1 and Par6 a

We tested syndecan-4 whether it was pulled down by the Rac1/CDC 42-binding domain of PAK (PAK-PKB) used in the Rac1 assay. The syndecan-4-GFP monomers were detected as a 55 kDa relative electrophoretic mobility band probed with anti-syndecan-4 or anti-GFP antisera. (Fig. 6). The amount of pulled-down syndecan-4 was in accordance with Rac1 level detected in the different syndecan-4 constructs (Fig. 6). The Ser179Ala syndecan-4 was preferred in the pulled-down experiment approximately 20 % more than the wt-syndecan-4, and there was only hardly detectable the Se179Glu syndecan-4 (Fig. 6). When γ-S GTP was applied to the cell lysates to equalize the pulled-down Rac1-GTP level there was no difference in the amount of pulled-down syndecan-4 variants (Fig. 6). The GDP loaded sample that is considered as negative control could not pull down active Rac1 neither syndecan-4 (data not shown). In direct experiment Rac1 co-precipitated with syndecan-4 (data not shown).

PDZ domain of Tiam-1 was reported to bind peptides containing aminoacid sequence of FYA (Songyang et al., 1997) that is part of the cytoplasmic segment of syndecan-4. Therefore syndecan-4 seemed a good candidate to interact with Tiam-1. In the immunoprecipitate of Tiam-1 syndecan-4 variants were detected by anti-syndecan-4 or anti-GFP antisera at 55 kDa length, which was correspondent to the monomer forms. There was no significant difference in the binding of Tiam-1 and syndecan-4 variants in the GDP or γ-S GTP saturated cases.

Considering that phospho-syndecan-4 down-regulates the Rac1 activity consequently Rac1 was also tested in the Tiam-1 precipitate to study the nature of inhibition. If the Rac1 activity were regulated by direct interaction of syndecan-4 we might expect a trapped intermedier as it was found in the case of Brefeldin A (Renault et al., 2003). Since Rac1 was observed in the wt- and Ser179Ala syndecan-4 but was missing in the Ser179Glu case so we concluded that phospho-syndecan-4 can block the Rac1-GDP binding to Tiam-1.

The Par polarity complex (Par6-Par3-aPKC) is known to control the epithelial polarization in a Racl/Cdc42 binding fashion. Recent reports now show that Tiam-1 regulates TJ assembly by binding to Par complex (Mertens et al., 2003). Par6 a, a component of the polarity complex having PDZ domains, and syndecan-4 was tested in a pull-down assay with anti-syndecan-4 or anti-Par6. All of the syndecan-4 variants were co-immunoprecipitated by anti-Par6, and Par6 by anti-syndecan-0, as well. The mutual co-immunoprecipitation provided evidence that syndecan-4 interacts with Par6. This interaction was GDP - GTP independent.

Taking together, syndecan-4 must interact with the Par polarity complex, since it was co-immunoprecipitated with Tiam-1 and Par6. The syndecan-4 pulldown by PAK-PKB resin is GTP-GDP - dependent, which supposes a Rac-effector protein interacting with syndecan-4.

### Example 11: Syndecan-4 influences the distribution of Tiam-1 and Par6 in MCF-7 in a Ser179 phosphorylation dependent manner

The *in vivo* distribution of Tiam-1 was studied in the cells expressing the different syndecan-4 variants. Distribution of Tiam-1 showed cytoskeleton-like orientation. Double labeled staining was employed for revealing the nature of Tiam-1 distribution. Microtubules were tested with anti-α-tubulin, however it co-localized with Tiam-1 just partially, the rest of the Tiam-stained filaments were different from the microtubulus. Intermediate filament cytokeratin was probed with pan-anti-cytokeratin. In the perinuclear region there was strong co-localization between Tiam-1 and cytokeratin, however in the edges of the cells co-distribution was not characteristic.

The Tiam-highlighted cytoskeletal pattern was monitored in the different cell lines. Sharp filaments were observed in the wt-, Ser179Ala expressing cells. In the Ser179Glu cells Tiam-1 was accumulated in the perinuclear region, although the anti-Tiam-1 visualized filamentous pattern was still seen at the cell edges. In migratory cells Tiam-1 concentrated mostly in the perinuclear region and at the trailing edges and less in the lamellipodia. However there was change in syndecan-4-knock down cells expressing shRNA syndecan-4. The anti-Tiam-1 immunostaining failed to visualize any structure, instead a strong perinuclear accumulation was observed in the syndecan-4 knocked down cells. The Rac1 was distributed homogeneously in the wt,- Ser179Ala syndecan-4 expressing cells and hardly co-localized with Tiam1, contrary to the syndecan-4 knock down cells, where Rac1 was co-localized with Tiam-1 mostly in the perinuclear region (Fig). The originally homogeneous distribution of Rac1 showed a massive perinuclear, and cell-peripheral accumulation in the Ser179Glu cells without any particular co-localization.

However, Par6a was co-distributed abundantly with all syndecan-4 variants mostly in the cytoplasm. Although Ser179Ala localized in the plasma membrane Par6a was hardly detected there, instead in the perinuclear region. In the Ser179Glu cells the accumulation of Par6a was similar to that of syndecan-4, it was concentrated mostly in the trailing edges.

Taking together, in the presence of non-phosphorylated syndecan-4 Tiam-1 showed a cytoskeletal distribution, that was changed in the Ser179Glu cell and was completely unstructured in the syndecan-4 knock down cells. Tiam-1 moved and Par6a co-localized with syndecan-4, meaning that syndecan-4 mediates the distribution of Tiam-1 and Par6a in a phosphorylation-dependent manner.

### Example 12: Short mutated peptides induce neuronal differentiation

Short peptides with the sequence RMKKKDEGEYDLG (SEQ.ID.NO.: 3) and RMKKKDEGDYDLG (SEQ.ID.NO.: 4) were tested under serum starvation. The cell responses shown on Fig. 8 were detected after 4-5 weeks, showing clear signs of the development of neuron-like phenotypes and the appearance of bIII-tubilin, which is a commonly used marker for neuronal differentiation.

### References

Adams JC. Molecular organisation of cell-matrix contacts: essential multiprotein assemblies in cell and tissue function. Expert Rev Mol Med. 2002 Feb 11;4(1):1-24.
Baciu PC, Goetinck PF. Protein kinase C regulates the recruitment of syndecan-4 into focal contacts. Mol Biol Cell. 1995 Nov;6(11):1503-13.
Bass MD, Humphries MJ. Cytoplasmic interactions of syndecan-4 orchestrate adhesion receptor and growth factor receptor signalling. Biochem J. 2002 Nov 15;368(Pt 1):1-15. Review.
Bass MD, Roach KA, Morgan MR, Mostafavi-Pour Z, Schoen T, Muramatsu T, Mayer U, Ballestrem C, Spatz JP, Humphries MJ. Syndecan-4-dependent Rac1 regulation determines directional migration in response to the extracellular matrix. J Cell Biol. 2007 May 7;177(3):527-38.
Bernfield M, Kokenyesi R, Kato M, Hinkes MT, Spring J, Gallo RL, Lose EJ. Biology of the syndecans: a family of transmembrane heparan sulfate proteoglycans. Annu Rev Cell Biol. 1992;8:365-93. Review. No abstract available.
Bloom L, Ingham KC, Hynes RO. Fibronectin regulates assembly of actin filaments and focal contacts in cultured cells via the heparin-binding site in repeat III13. Mol Biol Cell. 1999 May;10(5):1521-36.
Carey DJ. Syndecans: multifunctional cell-surface co-receptors. Biochem J. 1997 Oct 1;327 (Pt 1):1-16. Review.
Couchman JR. Syndecans: proteoglycan regulators of cell-surface microdomains? Nat Rev Mol Cell Biol. 2003 Dec;4(12):926-37.
Couchman JR, Vogt S, Lim ST, Lim Y, Oh ES, Prestwich GD, Theibert A, Lee W, Woods A. Regulation of inositol phospholipid binding and signaling through syndecan-4. J Biol Chem. 2002 Dec 20;277(51):49296-303. Epub 2002 Oct 10.
Chaudhuri P, Colles SM, Fox PL, Graham LM. Protein kinase Cdelta-dependent phosphorylation of syndecan-4 regulates cell migration. Circ Res. 2005 Sep 30;97(7):674-81. Epub 2005 Sep 1.
DesMarais V, Ghosh M, Eddy R, Condeelis J. Cofilin takes the lead. J Cell Sci. 2005 Jan 1;118(Pt 1):19-26.
Dovas A, Yoneda A, Couchman JR. PKCbeta-dependent activation of RhoA by syndecan-4 during focal adhesion formation. J Cell Sci. 2006 Jul 1;119(Pt 13):2837-46.
Elfenbein Al, Rhodes JM, Meller J, Schwartz MA, Matsuda M, Simons M, Suppression of RhoG activity is mediated by a syndecan 4-synectin-RhoGDII complex and is reversed by PKCalpha in a Rac activation pathway. J Cell Biol. 2009 Jul 13:186(1):75-83.
Ethell IM, Irie F, Kalo MS, Couchman JR, Pasquale EB, Yamaguchi Y. EphB/syndecan-2 signaling in dendritic spine morphogenesis. Neuron. 2001 Sep 27;31(6):1001-13.
Friedl P, Hegerfeldt Y, Tusch M. Collective cell migration in morphogenesis and cancer. Int J Dev Biol. 2004;48(5-6):441-9.
Greene DK, Tumova S, Couchman JR, Woods A. Syndecan-4 associates with alpha-actinin. J Biol Chem. 2003 Feb 28;278(9):7617-23.
Horowitz A, Simons M. Phosphorylation of the cytoplasmic tail of syndecan-4 regulates activation of protein kinase Calpha. J Biol Chem. 1998 Oct 2;273(40):25548-51.
Horowitz A, Simons M. Regulation of syndecan-4 phosphorylation in vivo. J Biol Chem. 1998 May 1;273(18):10914-8.
Hu K, Ji L, Applegate KT, Danuser G, Waterman-Storer CM. Differential transmission of actin motion within focal adhesions. Science. 2007 Jan 5;315(5808):111-5.
Hynes RO. Integrins: bidirectional, allosteric signaling machines. Cell. 2002 Sep 20;110(6):673-87. Review. Hynes RO. A reevaluation of integrins as regulators of angiogenesis. Nat Med. 2002 Sep;8(9):918-21. Review. Ishiguro K, Kadomatsu K, Kojima T, Muramatsu H, Tsuzuki S, Nakamura E, Kusugami K, Saito H, Muramatsu T. Syndecan-4 deficiency impairs focal adhesion formation only under restricted conditions. J Biol Chem. 2000 Feb 25;275(8):5249-52.
Itoh RE, Kurokawa K, Ohba Y, Yoshizaki H, Mochizuki N, Matsuda M. Activation of rac and cdc42 video imaged by fluorescent resonance energy transfer-based single-molecule probes in the membrane of living cells. Mol Cell Biol. 2002 Sep;22(18):6582-91.
Iwabu A, Smith K, Allen FD, Lauffenburger DA, Wells A. Epidermal growth factor induces fibroblast contractility and motility via a protein kinase C delta-dependent pathway. J Biol Chem. 2004 Apr 9; 279(15):14551-60. Epub 2004 Jan 27. Keum E, Kim Y, Kim J, Kwon S, Lim Y, Han I, Oh ES. Syndecan-4 regulates localization, activity and stability of protein kinase C-alpha. Biochem J. 2004 Mar 15;378(Pt 3):1007-14.
Kharait S, Dhir R, Lauffenburger D, Wells A. Protein kinase Cdelta signaling downstream of the EGF receptor mediates migration and invasiveness of prostate cancer cells. Biochem Biophys Res Commun. 2006 May 12;343(3):848-56..
Kokenyesi R, Bernfield M. Core protein structure and sequence determine the site and presence of heparin sulfate and chondroitin sulfate on syndecan-1. J Biol Chem. 1994 Apr 22;269(16):12304-9.
Koo BK, Jung YS, Shin J, Han I, Mortier E, Zimmermann P, Whiteford JR, Couchman JR, Oh ES, Lee W. Structural basis of syndecan-4 phosphorylation as a molecular switch to regulate signaling. J Mol Biol. 2006 Jan 27;355(4):651-63.
Kuriyama S, Mayor R. A role for Syndecan-4 in neural induction involving ERK-and PKC-dependent pathways. Development. 2009 Feb;136(4):575-84.
Langford JK, Stanley MJ, Cao D, Sanderson RD. Multiple heparan sulfate chains are required for optimal syndecan-1 function. J Biol Chem. 1998 Nov 6;273(45):29965-71.
Lim ST, Longley RL, Couchman JR, Woods A. Direct binding of syndecan-4 cytoplasmic domain to the catalytic domain of protein kinase C alpha (PKC alpha) increases focal adhesion localization of PKC alpha. J Biol Chem. 2003 Apr 18;278(16):13795-802.
Linseman DA, Loucks FA. Diverse roles of Rho family GTPases in neuronal development, survival, and death. Front Biosci. 2008 Jan 1;13:657-76.
Longley RL, Woods A, Fleetwood A, Cowling GJ, Gallagher JT, Couchman JR. Control of morphology, cytoskeleton and migration by syndecan-4. J Cell Sci. 1999 Oct;112 (Pt 20):3421-31.
Matthews HK, Marchant L, Carmona-Fontaine C, Kuriyama S, Larrain J, Holt MR, Parsons M, Mayor R. Directional migration of neural crest cells in vivo is regulated by Syndecan-4/Rac1 and non-canonical Wnt signaling/RhoA. Development. 2008 May;135(10):1771-80.
Mertens AE, Roovers RC, Collard JG. Regulation of Tiam1-Rac signaling, FEBS Letters, 2003 Jul 3, 546(1):11-16.
Murakami M, Horowitz A, Tang S, Ware JA, Simons M. Protein kinase C (PKC) delta regulates PKCalpha activity in a Syndecan-4-dependent manner. J Biol Chem. 2002 Jun 7;277(23):20367-71.
Murakami M, Elfenbein A, Simons M. Non-canonical fibroblasts growth factor signalling in angiogenesis. Cardiovasc. Res. 2008 78(2): 223-231.
Mostafavi-Pour Z, Askari JA, Parkinson SJ, Parker PJ, Ng TT, Humphries MJ. Integrin-specific signaling pathways controlling focal adhesion formation and cell migration. J Cell Biol. 2003 Apr 14;161(1):155-67. Nelson WJ. Epithelial cell polarity from the outside looking in. News Physiol Sci. 2003 Aug;18:143-6..
Ng T, Shima D, Squire A, Bastiaens PI, Gschmeissner S, Humphries MJ, Parker PJ. PKCalpha regulates betal integrin-dependent cell motility through association and control of integrin traffic. EMBO J. 1999 Jul 15;18(14):3909-23.
Nobes CD, Hall A. Rho, rac, and cdc42 GTPases regulate the assembly of multimolecular focal complexes associated with actin stress fibers, lamellipodia, and filopodia. Cell. 1995 Apr 7;81(1):53-62.
Noren NK, Niessen CM, Gumbiner BM, Burridge K. Cadherin engagement regulates Rho family GTPases. J Biol Chem. 2001 Sep 7;276(36):33305-8. Epub 2001 Jul 16.
Oh ES, Woods A, Couchman JR. Multimerization of the cytoplasmic domain of syndecan-4 is required for its ability to activate protein kinase C. J Biol Chem. 1997 May 2;272(18):11805-11.
Oh ES, Woods A, Couchman JR. Syndecan-4 proteoglycan regulates the distribution and activity of protein kinase C. J Biol Chem. 1997 Mar 28;272(13):8133-6.
Ohta Y, Hartwig JH, Stossel TP. FilGAP, a Rho- and ROCK-regulated GAP for Rac binds filamin A to control actin remodelling. Nat Cell Biol. 2006 Aug;8(8):803-14. Epub 2006 Jul 23.
Parsons M, Keppler MD, Kline A, Messent A, Humphries MJ, Gilchrist R, Hart IR, Quittau-Prevostel C, Hughes WE, Parker PJ, Ng T. Site-directed perturbation of protein kinase C- integrin interaction blocks carcinoma cell chemotaxis. Mol Cell Biol. 2002 Aug;22(16):5897-911.
Ridley AJ, Schwartz MA, Burridge K, Firtel RA, Ginsberg MH, Borisy G, Parsons JT, Horwitz AR. Cell migration: integrating signals from front to back. Science. 2003 Dec 5;302(5651):1704-9.
Ridley AJ. Pulling back to move forward. Cell. 2004 Feb 6;116(3):357-8.
Ridley AJ. Rho GTPases and actin dynamics in membrane protrusions and vesicle trafficking. Trends Cell Biol. 2006 Oct;16(10):522-9.
Saoncella S, Calautti E, Neveu W, Goetinck PF. Syndecan-4 regulates ATF-2 transcriptional activity in a Rac1-dependent manner. J Biol Chem. 2004 Nov 5;279(45):47172-6. Epub 2004 Sep 13
Saoncella S, Echtermeyer F, Denhez F, Nowlen JK, Mosher DF, Robinson SD, Hynes RO, Goetinck PF. Syndecan-4 signals cooperatively with integrins in a Rho-dependent manner in the assembly of focal adhesions and actin stress fibers. Proc Natl Acad Sci USA. 1999 Mar 16;96(6):2805-10.
Sander EE, ten Klooster JP, van Delft S, van der Kammen RA, Collard JG. Rac downregulates Rho activity: reciprocal balance between both GTPases determines cellular morphology and migratory behavior. J Cell Biol. 1999 Nov 29;147(5):1009-22.
Small JV, Rottner K, Kaverina I, Anderson KI. Assembling an actin cytoskeleton for cell attachment and movement. Biochim Biophys Acta. 1998 Sep 16;1404(3):271-81. Review.
Timar J, Raso E, Fazakas ZS, Silletti S, Raz A, Honn KV. Multiple use of a signal transduction pathway in tumor cell invasion. Anticancer Res. 1996 Nov-Dec;16(6A):3299-306.
Tkachenko E, Lutgens E, Stan RV, Simons M. Fibroblast growth factor 2 endocytosis in endothelial cells proceed via syndecan-4-dependent activation of Rac1 and a Cdc42-dependent macropinocytic pathway. J Cell Sci. 2004 Jul 1;117(Pt 15):3189-99.
Torka R, Thuma F, Herzog V, Kirfel G. ROCK signaling mediates the adoption of different modes of migration and invasion in human mammary epithelial tumor cells. Exp Cell Res. 2006 Nov 15;312(19):3857-71.
Torricelli C, Fortino V, Capurro E, Sacchi G, Ponzo P, Pacini A, Muscettola M, Maioli E. Role of PTHrp and PTHrp-engaged pathways in MCF-7 cells migration/invasion. Matrix Biol. 2006 Mar;25(2):104-11. Epub 2006 Feb 7.
Yonemura S, Itoh M, Nagafuchi A, Tsukita S. Cell-to-cell adherens junction formation and actin filament organization: similarities and differences between non-polarized fibroblasts and polarized epithelial cells. J Cell Sci. 1995 Jan;108 (Pt 1):127-4.
Wilcox-Adelman SA, Denhez F, Goetinck PF. Syndecan-4 modulates focal adhesion kinase phosphorylation. J Biol Chem. 2002 Sep 6;277(36):32970-7. Epub 2002 Jun 26.
Woods A, Couchman JR. Syndecan-4 and focal adhesion function. Curr Opin Cell Biol. 2001 Oct;13(5):578-83.
Woods A, Couchman JR. Syndecan 4 heparan sulfate proteoglycan is a selectively enriched and widespread focal adhesion component. Mol Biol Cell. 1994 Feb;5(2):183-92.
Zamir E, Geiger B. Molecular complexity and dynamics of cell-matrix adhesions. J Cell Sci. 2001 Oct;114(Pt 20):3583-90. Review.
Zamir E, Geiger B. Components of cell-matrix adhesions. J Cell Sci. 2001 Oct;114(Pt 20):3577-9. No abstract available.
Zhang J, Betson M, Erasmus J, Zeikos K, Bailly M, Cramer LP, Braga VM. Actin at cell-cell junctions is composed of two dynamic and functional populations. J Cell Sci. 2005 Dec 1;118(Pt 23):5549-62. Ziegler WH, Liddington RC, Critchley DR. The structure and regulation of vinculin. Trends Cell Biol. 2006 Sep;16(9):453-60.

## Claims

1. Polypeptide selected from the group consisting of the full length human syndecan-4, the cytoplasmic domain of human syndecan-4, and a fragment of human syndecan-4 cytoplasmic domain comprising the serine residue at position 179 (Ser179) of at least 13 amino acids in length, wherein said Ser179 residue of syndecan-4 is in phosphorylated state or modified to a phosphomimetic amino acid residue, for use in the treatment of a disease selected from the group consisting of cancer, Down syndrome, Wiskott-Aldrich syndrome, leukemia, ischemia or excitotoxicity, polyglutamine diseases, traumatic nerve injury, amyotrophic lateral sclerosis, neuropsychiatric disorder and neurodevelopmental disorders, wherein said polypeptide decreases the activity of Rac1.

2. The polypeptide for the use according to claim 1, wherein the cancer is selected from the group consisting of breast adenocarcinoma, colon carcinoma, gastric carcinoma, prostate carcinoma, endometrial carcinoma, melanoma.

3. The polypeptide for the use according to claim 1, wherein the neuropsychiatric disorder is selected from the group consisting of schizophrenia, Alzheimer's disease, HIV encephalitis.

4. The polypeptide for the use according to claim 1, wherein the neurodevelopmental disorder is selected from the group consisting of fragile X mental retardation syndrome, tuberous sclerosis complex, autism, Rett syndrome, sympathetic neuron death.

5. The polypeptide for use according to any one of claims 1 to 4, wherein the polypeptide has the sequence of SEQ.ID.NO.: 3 or SEQ.ID.NO.: 4.

6. The polypeptide for use according to any one of claims 1 to 5, wherein said phosphomimetic amino acid residue is the result of a Ser179Glu modification.

7. Polypeptide selected from the group consisting of the full length human syndecan-4, the cytoplasmic domain of human syndecan-4, and a fragment of human syndecan-4 cytoplasmic domain comprising the serine residue at position 179 (Ser179) of at least 13 amino acids in length, wherein said Ser179 residue of syndecan-4 is in dephosphorylated state or modified to a phosphodefective amino acid residue, for use in the treatment of Chronic granulomatous disease (CGD), wherein said polypeptide increases the activity of Rac1.

8. The polypeptide for use according to claim 7, wherein the polypeptide has the sequence of SEQ.ID.NO.: 1 or SEQ.ID.NO.: 2.

9. The polypeptide for use according to claim 7 or 8, wherein said phosphodefective amino acid residue is the result of a Ser179Ala modification.

## Patentansprüche

1. Polypeptid, ausgewählt aus der Gruppe bestehend aus dem humanen Volllängen-Syndecan-4, der zytoplasmatischen Domäne des humanen Sydecan-4, und einem Fragment der humanen zytoplasmatischen Syndecan-4 Domäne, aufweisend den Serinrest bei Position 179 (Ser179) mit zumindest 13 Aminosäuren Länge, wobei der Ser179-Rest des Syndecan-4 im phosphorylierten Zustand oder zu einem phosphomimetischen Aminosäurerest modifiziert ist, zur Verwendung in der Behandlung einer Erkrankung, ausgewählt aus der Gruppe bestehend aus Krebserkrankung, Down-Syndrom, Wiskott-Aldrich-Syndrom, Leukämie, Ischämie oder Exzitotoxizität, Polyglutaminerkrankungen, traumatische Nervenschädigung, amyotrophe Lateralsklerose, neuropsychiatrische Störung und neurologische Entwicklungsstörungen, wobei das Polypeptid die Aktivität von Rac1 verringert.

2. Polypeptid zur Verwendung gemäß Anspruch 1, wobei die Krebserkrankung ausgewählt ist aus der Gruppe bestehend aus Brustadenokarzinom, Kolonkarzinom, Magenkarzinom, Prostatakarzinom, Endometriumkarzinom, Melanom.

3. Polypeptid zur Verwendung gemäß Anspruch 1, wobei die neuropsychiatrische Störung ausgewählt ist aus der Gruppe bestehend aus Schizophrenie, Alzheimer-Krankheit, HIV-Enzephalitis.

4. Polypeptid zur Verwendung gemäß Anspruch 1, wobei die neurologische Entwicklungsstörung ausgewählt ist aus der Gruppe bestehend aus Fragiles-X-Syndrom, tuberöse Sklerose, Autismus, RettSyndrom, symphathischer Neuronentod.

5. Polypeptid zur Verwendung nach einem der Ansprüche 1 bis 4, wobei das Polypeptid die Sequenz der SEQ.ID.NO.: 3 oder SEQ.ID.NO.: 4 hat.

6. Polypeptid zur Verwendung nach einem der Ansprüche 1 bis 5, wobei der phosphomimetische Aminosäurerest das Ergebnis einer Ser179Glu-Modifikation ist.

7. Polypeptid, ausgewählt aus der Gruppe bestehend aus dem humanen Volllängen-Syndecan-4, der zytoplasmatischen Domäne des humanen Sydecan-4, und einem Fragment der humanen zytoplasmatischen Syndecan-4 Domäne, aufweisend den Serinrest bei Position 179 (Ser179) mit zumindest 13 Aminosäuren Länge, wobei der Ser179-Rest des Syndecan-4 im dephosphorylierten Zustand oder zu einem unphosphorylierbaren Aminosäurerest modifiziert ist, zur Verwendung in der Behandlung von septischer Granulomatose (CGD), wobei das Polypeptid die Aktivität von Rac1 erhöht.

8. Polypeptid zur Verwendung gemäß Anspruch 7, wobei das Polypeptid die Sequenz der SEQ.ID.NO.: 1 oder SEQ.ID.NO.: 2 hat.

9. Polypeptid zur Verwendung gemäß Anspruch 7 oder 8, wobei der unphosphorylierbare Aminosäurerest das Ergebnis einer Ser179Ala Modifikation ist.

## Revendications

1. Polypeptide choisi parmi le groupe constitué par la syndécane-4 humaine de longueur totale, le domaine cytoplasmique de la syndécane-4 humaine et un fragment du domaine cytoplasmique de la syndécane-4 humaine comprenant le résidu de sérine à la position 179 (Ser179) possédant une longueur d'au moins 13 acides aminés, ledit résidu Ser179 de la syndécane-4 étant à l'état phosphorylé ou modifié pour obtenir un résidu d'acide aminé phosphomimétique, pour son utilisation dans le traitement d'une maladie choisie parmi le groupe constitué par un cancer, le syndrome de Down, le syndrome de Wiskott-Aldrich, la leucémie, une ischémie ou une excitotoxicité, des maladies par expansion de polyglutamine, des lésions nerveuses traumatiques, la sclérose latérale amyotrophique, des troubles neuropsychiatriques et des troubles neurologiques du développement, ledit polypeptide diminuant l'activité de Rac1.

2. Polypeptide pour son utilisation selon la revendication 1, dans lequel le cancer est choisi parmi le groupe constitué par l'adénocarcinome du sein, le carcinome du côlon, le carcinome gastrique, le carcinome de la prostate, le carcinome endométrial, un mélanome.

3. Polypeptide pour son utilisation selon la revendication 1, dans lequel le trouble neuropsychiatrique est choisi parmi le groupe constitué par la schizophrénie, la maladie d'Alzheimer, l'encéphalite à VIH.

4. Polypeptide pour son utilisation selon la revendication 1, dans lequel le trouble neurologique du développement est choisi parmi le groupe constitué par le syndrome de l'X fragile, la sclérose tubéreuse du cerveau, l'autisme, le syndrome de Rett, la mort des neurones sympathiques.

5. Polypeptide pour son utilisation selon l'une quelconque des revendications 1 à 4, dans lequel le polypeptide possède la séquence de la SEQ ID NO: 3 ou de la SEQ ID NO: 4.

6. Polypeptide pour son utilisation selon l'une quelconque des revendications 1 à 5, dans lequel ledit résidu d'acide aminé phosphomimétique est le résultat d'une modification Ser179Glu.

7. Polypeptide choisi parmi le groupe constitué par la syndécane-4 humaine de longueur totale, le domaine cytoplasmique de la syndécane-4 humaine et un fragment du domaine cytoplasmique de la syndécane-4 humaine comprenant le résidu de sérine à la position 179 (Ser179) possédant une longueur d'au moins 13 acides aminés, ledit résidu Ser179 de la syndécane-4 étant à l'état déphosphorylé ou modifié pour obtenir un résidu d'acide aminé phosphodéficient, pour son utilisation dans le traitement de la granulomatose septique chronique (CGD), ledit polypeptide augmentant l'activité de Rac1.

8. Polypeptide pour son utilisation selon la revendication 7, dans lequel le polypeptide possède la séquence de la SEQ ID NO: 1 ou de la SEQ ID NO: 2.

9. Polypeptide pour son utilisation selon la revendication 7 ou 8, dans lequel ledit résidu d'acide aminé phosphodéficient est le résultat d'une modification Ser179Ala.


### SEQUENCE LISTING

<110> SZILAK LABORATORIES BIOINFORMATICS & MOLECULE-DESIGN LTD.
<120> SYNDECAN-4 IS A REGULATOR OF RAC1-GTP
<130> 106081/LT
<160> 5
<170> PatentIn version 3.5
<210> 1
   <211> 53
   <212> PRT
   <213> Artificial sequence
<220>
   <223> Syndecan-4 sequence
<400> 1
   Val Leu Ala Ala Leu Ile Val Gly Gly Ile Val Gly Ile Leu Phe Ala
   1               5                   10                  15
   Val Phe Leu Ile Leu Leu Leu Met Tyr Arg Met Lys Lys Lys Asp Glu
               20                  25                  30
   Gly Ser Tyr Asp Leu Gly Lys Lys Pro Ile Tyr Lys Lys Ala Pro Thr
           35                  40                  45
   Asn Glu Phe Tyr Ala
       50
<210> 2
   <211> 13
   <212> PRT
   <213> Artificial Sequence
<220>
   <223> Syndecan-4 sequence
<400> 2
   Arg Met Lys Lys Lys Asp Glu Gly Ala Tyr Asp Leu Gly
   1               5                   10
<210> 3
   <211> 13
   <212> PRT
   <213> Artificial sequence
<220>
   <223> Syndecan-4 sequence
<400> 3
   Arg Met Lys Lys Lys Asp Glu Gly Glu Tyr Asp Leu Gly
   1               5                   10
<210> 4
   <211> 13
   <212> PRT
   <213> Artificial sequence
<220>
   <223> Syndecan-4 sequence
<400> 4
   Arg Met Lys Lys Lys Asp Glu Gly Asp Tyr Asp Leu Gly
   1               5                   10
<210> 5
   <211> 24
   <212> PRT
   <213> Artificial sequence
<220>
   <223> Syndecan-4 sequence
<400> 5
   Arg Met Lys Lys Lys Asp Glu Gly Ser Tyr Asp Leu Gly Lys Lys Pro
   1               5                   10                  15
   Ile Tyr Lys Lys Ala Pro Thr Asn
               20